# EUROPEAN PATENT SPECIFICATION

(11) **EP 0 630 249 B1**
(45) Date of publication and mention of the grant of the patent: **18.09.2002**
(21) Application number: 93906054.7
(22) Date of filing: 18.02.1993
(51) Int. Cl.: A61K 31/70, A61K 31/74, C07H 21/00

(54) **DUAL ACTION 2',5'-OLIGOADENYLATE ANTIVIRAL DERIVATIVES AND USES THEREOF**
ANTIVIRALE 2',5'-OLIGOADENYLATDERIVATE MIT DOPPELTER WIRKUNG UND IHRE VERWENDUNGEN
DERIVES ANTIVIRAUX DE 2'-5'-OLIGODENYLATE A DOUBLE ACTION ET LEURS UTILISATIONS

(30) Priority: 12.03.1992 US 849865
(43) Date of publication of application: 28.12.1994
(73) Proprietor: Temple University of the Commonwealth System of Higher Education, Philadelphia PA 19122 (US)
(72) Inventor: SUHADOLNIK, Robert J., Roslyn, PA (US); PFLEIDERER, Wolfgang, D-7750 Konstanz (DE)
(74) Representative: W.P. THOMPSON & CO.
(86) International application number: US9301446
(87) International publication number: WO93017692

(56) References cited:
- US-A- 4 464 359
- US-A- 4 924 624
- US-A- 4 981 957
- HELVETICA CHIMICA ACTA, vol. 74, 1991 BASEL CH, pages 887-891, S.MIKHAILOV ET AL. 'Synthesis of 3'-Deoxyadenylyl-(2'-5')-3'-deoxyadenylyl- (2'-w)-9-(w-hydroxyalkyl)adenines.'
- CHEMICAL ABSTRACTS, vol. 110, no. 15, 10 April 1989 Columbus, Ohio, US; abstract no. 131357j, P.F.TORRENCE ET AL. 'Substrates Resistant to 2'-5'-Phosphodiesterase Activity.' page 351; column 2; & US-A-72 666 (U.S. DEPARTMENT OF HEALTH AND HUMAN SERVICES) 1988
- CHEMICAL ABSTRACTS, vol. 104, no. 15, 14 April 1986 Columbus, Ohio, US; abstract no. 122647c, C.BISBAL ET AL. '2',5'-Oligoadenylate Analogs: Synthesis, Biological Activities and Intracellular Delivery.' page 26; column 1; & PROG. CLIN. BIOL. RES., vol. 202, 1985 pages 89-95,

## Description

### Field of the Invention

The invention relates to certain therapeutic 2',5'-oligoadenylate analogs, pharmaceutical conjugates and compositions of such analogs, and uses thereof.

### Background of the Invention

The full nomenclature of the subject matter of the present invention involves extremely long terms. It is customary for those skilled in the art to abbreviate these terms in a manner well known to them. These general and customary abbreviations are set forth herein below and may be utilized in the text of this specification.

### Abbreviations:

RT, reverse transcriptase
A, adenosine or adenylate or adenylyl
cordycepin or C or 3'-dA, 3'-deoxyadenosine-(3'-deoxyadenylate)
ara-A, 9-ß-D-arabinofuranosyladenine
EHNA, erthyro-9-(2-hydroxy-3-nonyl)adenine
A-3'-amino, 3'-amino-3'-deoxyadenosine
tubercidin, 4-amino-7(B-D-ribofuranosyl)-pyrrolo-[2,3-d]pyrimidine
3'-dATP, 3'-deoxyadenosine triphosphate
ATP, adenosine triphosphate
I, inosine or inosinate or inosinylyl
Xylo-A or xyloadenosine, 9-β-D-xylofuranosyladenine
dCF or 2'-deoxycoformycin, (R)-3-(2-deoxy-β-D-erythropentofuranosyl)-3,6,7,8-tetrahydroimidazo[4,5-d][1,3]diazepine-8-ol
2-5A or 2',5'-oligo(A) or 2',5'-oligoadenylate, oligomer of adenylic acid with 2',5'-phosphodiester linkages and a triphosphate at the 5'-end
C, cordecypin
2',5'-cordycepin analog or 2',5'-oligocordycepin, oligomer of 3'-deoxyadenylic acid with 2',5'-phosphodiester linkages and a triphosphate at the 5'-end
2',5'-Aₙ or core oligomer, oligomer of adenylic acid with 2',5'-phosphodiester linkages
2',5'-A₃ or 2',5'-adenylate trimer core, adenylyl(2',5')adenylyl(2',5')adenosine
2',5'-A₄ or 2',5'-adenylate tetramer core, adenylyl(2',5')adenylyl(2',5')adenylyl(2',5')adenosine
2',5'-3'dA₃ or 2',5'-C-C-C or 2',5'-cordycepin trimer core, 3'-deoxyadenylyl(2',5')3'-deoxyadenylyl-(2',5')3'-deoxyadenosine
2',5'-C-C-C-C or 2',5'-cordycepin tetramer core,3'-deoxyadenylyl(2',5')3'-deoxyadenylyl(2',5')3'-deoxyadenylyl(2',5')3'-deoxyadenosine
3',5'-A₃, adenylyl(3',5')adenylyl(3',5')adenosine
2',5'-I₃ or 2',5'-inosine trimer core, inosinylyl(2',5')inosinylyl(2',5')inosine
dd benz, benzimidazylyl(2',5')5,6-dichlorobenzimidazole riboside,
EBV, Epstein-Barr virus
EBNA, Epstein-Barr virus associated early nuclear antigen
HBV, hepatitis B virus
HIV, human immunodeficiency virus, including HIV-1, HIV-2, and all other HIV subtypes
HBLV, human B-cell lymphotropic virus
HTLV, human T-cell leukemia virus, including HTLV-I, HTLV-II and HTLV-III, and all other HTLV subtypes
IFNα: α-interferon
rIFN-αA: recombinant α-interferon
dsRNA: double-strand ribonucleic acid
2',5'-A-A-Tu, adenylyl(2',5')adenylyl(2',5')-tubercidin
2',5'-Tu-Tu-Tu, 2',5'-tubercidylyl(2',5')-tubercidylyl(2',5')tubercidin
2',5'-A-A-ara-A, adenylyl(2',5')adenylyl-(2',5')ara-A
2',5'-C-C-A, 3'-deoxyadenylyl(2',5')3'-deoxyadenylyl(2',5')adenosine
2',5'-A-C-C, adenylyl(2',5')3'-deoxyadenylyl-(2',5')3'-deoxyadenosine
2',5'-A-A-C,adenylyl(2',5')adenylyl(2',5')3'-deoxyadenosine
2',5'-C-A-C, 3'-deoxyadenylyl(2',5')adenylyl-(2',5')3'-deoxyadenosine
2',5'-C-C-A, 3'-deoxyadenylyl(2',5')3'-deoxyadenylyl(2',5')adenosine
2',5'-A-C-A, adenylyl(2',5')3'-deoxyadenylyl-(2',5')adenosine
2',5'-xylo-A₃, xyloadenylyl(2',5')xyloadenylyl-(2',5')xyloadenosine
2',5'-xylo-A₄, xyloadenylyl(2',5')xyloadenylyl-(2',5')xyloadenylyl(2',5')xyloadenosine
Ac, acetyl
Bz, benzyl
MMTr, 5'-0-p-methoxytrityl
2',5'-trityl-C₃, 5'-0-p-methoxytrityl-3'-deoxyadenylyl(2',5')3'-deoxyadenylyl(2',5')3'-deoxyadenosine
2',5'-trityl-A₃, 5'-0-p-methoxytrityladenylyl-(2',5')adenylyl(2',5')adenosine
2',5'-C-C-dCF, 3'-deoxyadenylyl(2',5')3'-deoxyadenylyl(2',5')2'-deoxycoformycin
2',5'-A-A-A-3'-amino,adenylyl(2',5')adenylyl-(2',5')3'-amino-3'-deoxyadenosine
SiTBD, t-butyldimethylsilyl or -Si (CH₃)₂C(CH₃)₃
2',5'-A₍ₛᵢ₎-A₍ₛᵢ₎-A, 3'-0-t-butyldimethylsilyladenylyl(2',5')3'-0-t-butyldimethylsilyladenylyl(2',5')-adenosine
2',5'-A-A-A-3'-0-methyl,adenylyl(2',5')adenylyl(2',5')3'-0-methyladenosine
2',5'-A-A-A-3'-0-pentyl,adenylyl(2',5')adenylyl(2',5')3'-0-pentyladenosine
2',5'-A-A-A-3'-0-hexyl, adenylyl(2',5')adenylyl(2',5')3'-0-hexyladenosine
2',5'-A-A-A-3'-0-heptyl,adenylyl(2',5')adenylyl(2',5')3'-0-heptyladenosine
2',5'-EHNA-A-A, erythro-9-(2-hydroxy-3-nonyl)adenylyl(2',5')adenylyl(2',5')adenosine

The abbreviation for the "tetramer" compounds comprising the adenylyl (A) and 3'deoxyadenylyl (C) moieties is illustrated by the following:
2',5'-A-A-C-C,adenylyl(2',5')adenylyl(2',5')-3'-deoxyadenylyl(2',5')3'-deoxyadenosine

The above compounds are also abbreviated without the 2'-5' prefix, without hyphens and without the -3' suffix; hence 2'-5'-C-C-C-3' is also abbreviated CCC.

With the expansion of the knowledge of the antiviral state induced by interferon, attention has been focused on the chemical and enzymatic synthesis and biological properties of the 2',5'-oligoadenylates as mediators of the antiretroviral response. 2',5'-Oligo-(A) is a component of a natural, broad-spectrum antiviral defense mechanism in plants and animals. The 2-5A pathway, also known as the 2-5A/RNase L pathway or antiviral system, is widely accepted to be involved in the antiviral mechanism of interferon, and is also involved in the regulation of cell growth and differentiation.

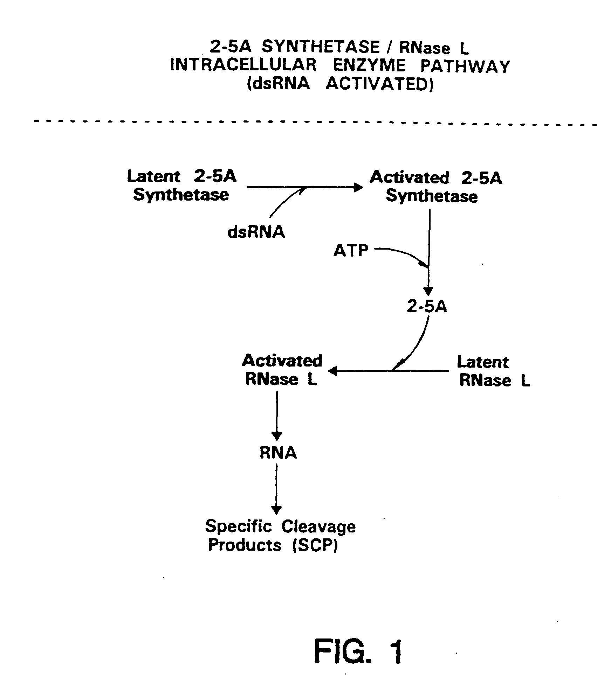

The pathway involves the activation by 2-5A of the latent endoribonuclease, RNase L (EC 3.1.27). According to that pathway shown in Fig. 1, 2-5A is synthesized from ATP by 2',5'-oligoadenylate synthetase [ATP: (2'-5')oligo(A)-adenyltransferase (EC 2.7.7.19)], hereinafter "2-5A synthetase". When activated by dsRNA, 2-5A synthetase converts ATP into 2-5A, i.e., a series of 2',5'-linked oligoadenylates characterized by a 5'-terminal triphosphate. 2-5A Synthetase exists in different isoenzyme forms, is induced by interferon, but is also detectable at lower levels in the absence of interferon. 2-5A exerts its biological effects by binding to and activating its only known target enzyme, the unique 2-5A dependent endoribonuclease RNase L. The latter cleaves viral and cellular mRNA or rRNA, thereby inhibiting protein synthesis. Hovanessian et al., Eur. J. Biochem. 93:515-526 (1979); Kerr et al., Proc. Natl. Acad. Sci. USA 75:256-260 (1978). The short half-life of the authentic 2-5A molecule in biological systems is an acknowledged disadvantage in the control of viral replication. Moreover, bioactive 2-5A is inactivated by three enzymes: a relatively unspecific 2'-phosphodiesterase, a 5'-phosphatase, and a relatively specific 2',3'-exonuclease. Some cytokines, e.g. IL-6, activate 2-5A synthetase in such a way as to cause the enzyme or particular forms of the enzyme to produce bioinactive forms of 2-5A (Bickel, M., Dveksler, G, Dieffenbach, C., W., Ruhl, S., Midura, S., B. and Pluznik, D.H., Cytokine 2:238-246 (1990) and Cohen, B., Gothelf, Y., Vaiman, D., Revel, M. and Chebath, J., Cytokine 3:83-91 (1991)).

The 2-5A synthetase/RNase L pathway is activated following viral infection by many viruses including HIV-1 (Schröder, H.C., Wenger, R., Kuchino, Y., and Müller, W.E.G., J. Biol. Chem. 264, 5669 (1989); Schröder, H.C., Wenger, R., Rottman, M., and Müller, W.E.G., Biol. Chem. Hoppe-Seyler 369, 985 (1988)). The activation of this pathway delays the HIV infection process. To activate RNase L, the naturally occurring 2-5A molecule requires a 5'-triphosphate, which is unstable. 2-5A molecules with 5'-monophosphates or no 5'-phosphate (core) do not activate RNase L at physiological concentrations.

"Human B-lymphotropic virus", also known as "human B-cell lymphotropic virus" (HBLV), now called HHV-6, which is characterized by a large molecular weight double-stranded DNA genome is morphologically similar to viruses of the herpes virus family, but is readily distinguishable from the known human and non-human primate herpes viruses by host range, in vitro biological effects, antigenic features and genome. Salahuddin et al., Science 234:596-601 (1986); Josephs et al., Science 234:601-602 (1986). The virus has been observed to selectively infect freshly isolated human B-cells, which are converted into large, refractile mono- or binucleated cells with nuclear and cytoplasmic inclusion bodies. HBLV is suspected to be the cause of a chronic mononucleosis-like syndrome characterized by chronic fatigue lasting more than a year.

Human immunodeficiency virus ("HIV"), also known as human T-cell leukemia virus III ("HTLV-III"), the etiologic agent of acquired immune deficiency syndrome, is a type D retrovirus. As in all retroviruses, an essential feature of HIV replication is reverse transcription of the plus-strand RNA genome into DNA, a process which requires an RNA dependent DNA polymerase, reverse transcriptase. This enzyme is viral-encoded and is found associated with genomic RNA in mature HIV virions. The exclusiveness of reverse transcriptase to retroviruses and viruses requiring a short reverse transcription step makes reverse transcriptase a major target for antiviral, and particularly for antiretroviral, therapeutic intervention.

The 2-5A synthetase/RNase L system as an antiviral cellular defense mechanism has been shown to be a promising target for antiviral chemotherapy, particularly due to its interaction with double-stranded segments within viral genomes or transcripts such as the HIV-1 RMA genome (Lengyel, P., Annu. Rev. Biochem. 51, 251 (1982); Pestka, S., ed., Methods Enzymol. 118, 119 (1986); Lengyel, P., J. Interferon Res. 7, 511 (1987); Sen, G.C., Prog. Nucleic Acid Res. Molec. Biol. 27, 105 (1982)). However, what is needed are derivatives of 2-5A which will override degradation by enzymes which inactivate authentic 2-5A. What is needed is .a method for controlling HIV, chronic fatigue caused by HBLV, and other vir or cytokine-induced disease states characterized by a 2-5A pathway defect using compounds that are more metabolically stable and active than authentic 2-5A. What is needed is a method for treatment of viral infection which utilizes compounds which have broad spectrum, dual action, that is, compounds which both activate the 2-5A pathway and inhibit the activity of viral DNA polymerase.

### Summary of the Invention

According to one embodiment, the invention comprises a novel compound of Formula I 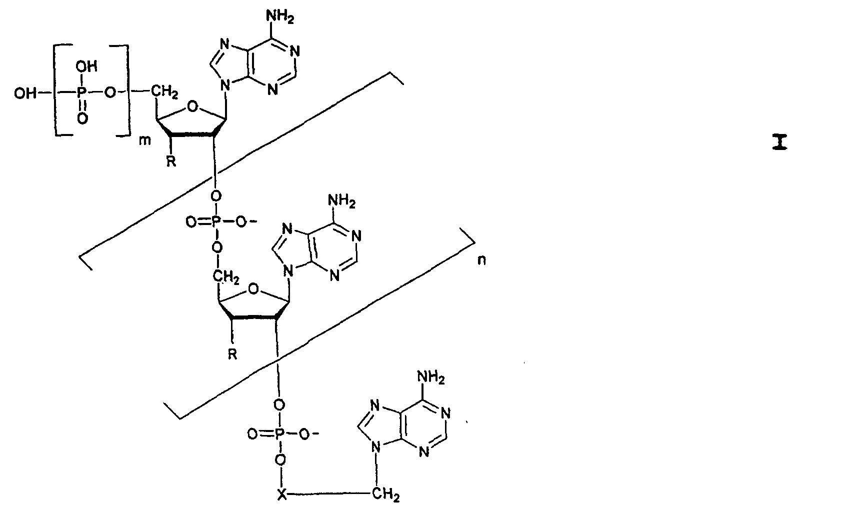 wherein
n is a whole positive integer from 1 to 8,
m is 0, 1, 2 or 3,
R is hydrogen,
X is selected from C₁ to C₆ alkylene and C₁ to C₆ alkyleneoxy, or
a pharmaceutically acceptable salt thereof.

Preferably n is 1 to 3, most preferably 1 or 2. X is preferably selected from C₁ to C₆ alkylene and C₁ to C₃ alkyleneoxy.

Corresponding pharmaceutical compositions and first and second medical uses are also claimed, the second medical use being inhibition of viral in mammals.

According to another embodiment, the invention provides a conjugate comprising a compound according to Formula II 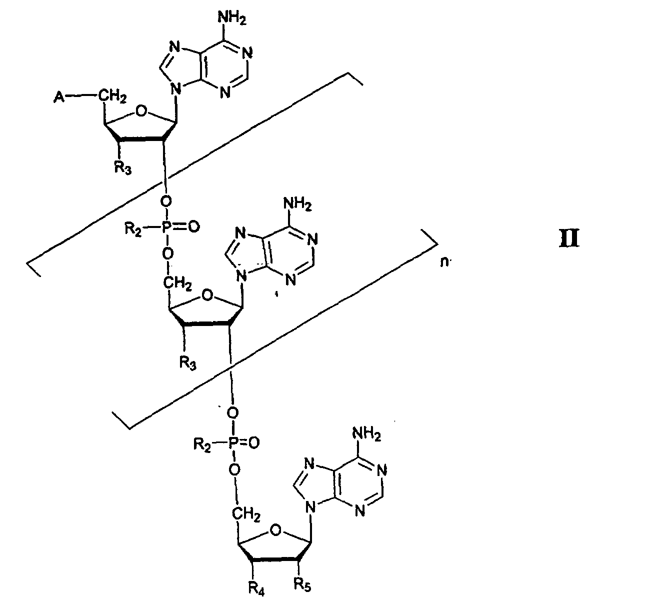 wherein
A is 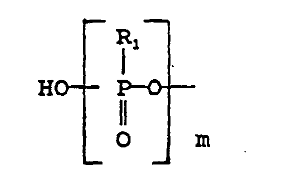 or 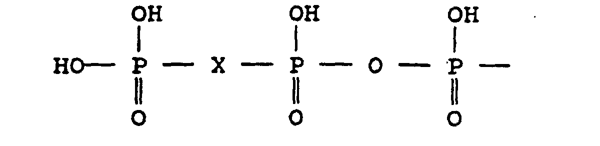 X is NH₂ or CH₂;
m is zero, 1, 2 or 3;
n is an integer from 1 to 8;
each R₁ is independently selected from O⁻, S⁻, sulfate, Se⁻, C₁ to C₈ alkyl and C₁ to C₈ alkoxy;
each R₂ is independently selected from O⁻, S⁻ , sulfate, Se, C₁ to C₈ alkyl and C₁ to C₈ alkoxy;
each R₃ is independently selected from hydrogen, hydroxyl, amino and -OSi(CH₃)₂-C(CH₃)₃;
R₄ and R₅ are independently selected from hydrogen; hydroxyl; amino; C₁ to C₈ alkyl; C₁ to C₈ alkoxy; C₁ to C₈ alkylamino, alkylcarbonyl, alkylcarboxyl and haloalkyl; and C₁ to C₈ alkoxyamino, alkoxycarbonyl, alkoxycarboxyl and haloalkoxy;
or pharmaceutically acceptable salts thereof, excluding authentic 2',5'-oligoadenylate and salts thereof, said compounds being covalently linked to an adduct through a hydroxyl oxygen at the 2'- or 3'- position of the 2'-terminal nucleotide of the compound, which adduct is selected from (i) vitamins which have corresponding cell surface receptors for receptor-mediated endocytosis, (ii) adducts containing the cholesteryl group, and (iii) acyl groups of the formula 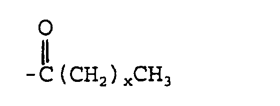 wherein x is an interger from 1 to 20, and which adduct results in enhanced penetration of the compound into intact cells.

Such conjugates, on administration, simultaneously cause activation of the 2-5A synthetase/RNase L antiviral pathway of said mammal and inhibition of viral DNA polymerase.

Preferably, n is 1 to 3, most preferably 1 or 2. According to yet another preference, the alkyl, alkoxy, substituted alkyl or substituted alkoxy groups, which may contain from one to 8 carbon atoms, preferably contain from 1 to 4 carbon atoms, most preferably from 1 to 3 carbon atoms. For halide-substituted alkyl and alkoxy groups, the halogen atom is preferably chlorine, bromine or fluorine, with chlorine being most preferred.

According to a preferred subgenus of the invention,
the group A is 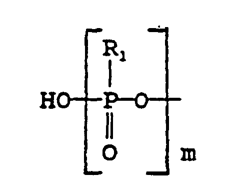 wherein m is defined above; and more preferably, at least one of R₁ are S⁻, where each other R₁ is O⁻. An additional preferred embodiment provides that at least one of R₃ or R₄ is hydrogen, the remainder being hydroxyl.

The adduct is most advantageously coupled to the oligomer through a hydroxyl oxygen at the 2' position of the 2'-terminal nucleotide, Vitamins (i) useful as adducts according to the present invention include, for example, vitamin B₁₂, biotin, riboflavin and folic acid.

When the adduct comprises a lipophilic molecule or radical, such as the acyl group (iii) of the formula 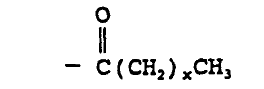 the adduct preferably contains a cholesteryl group.

According to one preferred subgenus of conjugate, the compound to which the adduct is conjugated has the structure of Formula II wherein
A is 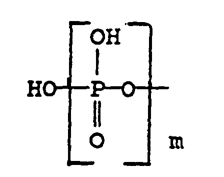 m is zero, 1, 2 or 3,
n is an integer from 1 to 8, preferably 1, 2 or 3,
each R₃ and R₄ is independently selected from hydrogen and hydroxyl,
each R₂ is independently selected from S⁻ and O⁻,
R₅ is hydroxy,
or a pharmaceutically acceptable salt thereof; which compound is covalently linked through the 2'-position of the 2'-terminal nucleotide thereof to the adduct.
In a further aspect, the invention provides a pharmaceutical composition comprising a pharmaceutical carrier and a compound according to formula I above wherein, in addition to the substitution possibilities set out above for formula I, R may be hydroxyl.

The invention also provides first and second medical uses of such compounds (except those in which R is hydrogen), the second medical use being inhibition of viral infection in mammals.

### Description of the Figures

Fig. 1 is a diagram illustrating the 2-5A synthetase/RNase L intracellular enzyme pathway.

Fig. 2A is a phase-contrast micrograph of H9 cells incubated for 5 hours with fluorescein-bovine serum albumin (BSA) covalently conjugated to folate (25 µg/ml) in folate-deficient medium.

Fig. 2B is a fluorescent micrograph of the same H9 cells shown in Fig. 2A.

Fig. 2C is a phase-contrast micrograph of H9 cells incubated for 5 hours with fluorescein-BSA not conjugated to folate.

Fig. 2D is a fluorescent micrograph of the same H9 cells shown in Fig. 2C.

### Detailed Description of the Invention

Therapeutic agents that activate the 2-5A synthetase/RNase L antiviral defense pathway and also inhibit the activity of virally-derived DNA polymers (particularly reverse transcriptase) are used for the treatment of viral disease states. By "treatment" is meant to include also prophylaxis. Such dual effect therapeutic agents are selective, broad spectrum inhibitors of viral DNA polymerases that are produced during viral infection. The polymerases are critical for viral replication. The compounds utilized in the practice of the invention may be characterized as derivatives of authentic 2-5A with modifications in the 5'-terminal phosphate group, ribosyl moiety and/or the internucleotide bonds. These derivatives are metabolically stable, non-toxic, and have dual antiviral effects, e.g., they activate the 2-5A synthetase/RNase L antiviral pathway and also inhibit virally-derived DNA polymerase. We have thus approached antiviral therapy by dually activating inherent antiviral mechanisms present in mammalian cells and inhibiting enzymes critical for viral information transfer.

According to one class of compounds useful in the practice of the invention, one or more of the 3'-hydroxyl groups was replaced by hydrogen atoms to form the core 2',5'-cordycepin derivatives. This modification resulted in derivatives with increased resistance to degradation by phosphodiesterase. The preparation of such compounds is disclosed in U.S. Patent 4,464,359 (2',5'-oligocordycepin), and in U.S. Patent 4,859,768 (2',5'-oligocordycepin and mixed 2',5'-oligo(cordycepin/adenosine)). The core 2',5'-cordycepin derivatives are also non-toxic and have broad spectrum antiretroviral activity in infected cells (Montefiori, D.C. Sobol, R.W., Li, S.W., Reichenbach, N.L., Suhadolnik, R.J., Charubala, R., Pfleiderer, W., Modliszewski, A., Robinson, W.E., Jr., and Mitchell, W.M., Proc. Natl. Acad. Sci. USA 86, 7191 (1989); Suhadolnik, R.J., Lebleu, B., Pfleiderer, W., Charubala, R., Montefiori, D.C., Mitchell, W.M., Sobol, R.W., Li, S.W., Kariko, K., and Reichenbach, N.L., Nucleosides & Nucleotides 8, 987 (1989); Müller, W.E.G., Weiler, B.E., Charubala, R., Pfleiderer, W., Leserman, L., Sobol, R.W., Suhadolnik, R.J., and Schröder, H.C., Biochemistry 30, 2027 (1991)). Other 2-5A derivatives have been synthesized with other modifications of the 3'-hydroxyl groups, such as the substitution of amino and OSi(CH₃)₂-C(CH₃)₃, as described in Patent 4,859,768. Other 2-5A derivatives contain, in addition to or in lieu of 3'-hydroxyl modifications on one or more nucleotides, modifications in the 5'-terminal phosphate group. These 2-5A derivatives retain the ability to inhibit viral DNA polymerase and retain metabolic stability but, in addition, are able to activate RNase L. A variety of 2'-5'-oligoadenylate derivatives in addition to those already synthesized will exhibit enhanced antiviral activity.

According to yet another embodiment, the internucleotide phosphodiester linkage is modified. According to a preferred embodiment, sulfur is substituted for an oxygen atom to form the 2',5'-phosphorothioate oligoadenylates. The preparation of such compounds, including optical isomers, is described in U.S.Patent 4,924,624. The substitution of sulfur for oxygen in the 2',5'-phosphodiester backbone introduceschirality into the molecules and introduces a new chemistry of the backbone. The core 2',5'-phosphorothioates exhibit increased resistance to phosphodiesterase and phosphatases and new biological activities compared to authentic 2-5A cores. These stereochemically modified molecules are the first 2',5'-linked core molecules able to activate RNase L. The 2',5'-phosphorothioates act through both the activation of the 2-5A synthetase/RNase L antiviral system and the inhibition of viral DNA polymerase. The 2',5'-phosphorothioate trimer 5'-monophosphates activate RNase L at nanomolar concentrations, similar to naturally occurring p₃A₃. Other molecular modifications of the internucleotide linkage provided for herein include the substitution of an oxygen atom by sulfate, selenium, C1-C8 alkyl and C1-C8 alkoxy.

In yet another embodiment, dual action, antiviral 2-5A derivatives having increased penetration into intact cells are prepared by conjugating the oligomer to an adduct. One preferred group of adducts comprise the water-soluble vitamins (including but not limited to biotin, folic acid, vitamin B₁₂, or riboflavin). Other preferred adducts include lipophilic molecular and chemical groups such as, for example, acyl or cholesteryl groups. Such conjugates are internalized by intact cells by exploiting receptor-mediated endocytosis.

Administration of exogenous, metabolically stable, dual action analogs of 2-5A will render increased protection against disorders characterized by a 2-5A defect, particularly protection against viral infection in animals and humans. By "2-5A defect" as used herein is meant any manifestation, condition or interruption of the 2-5A pathway which results in a physiologically consequential change in the production of authentic 2-5A, and/or the interruption of 2-5A-dependent activation of RNase L. Afflictions characterized by a 2-5A defect include, for example, viral infections, particularly HTLV infection, most particularly HIV infection, chronic fatigue and other HHV-6 related disorders, hepatitis B and other infections of the hepatitis virus family, cutaneous T-cell lymphoma and other HTLV-1 related disorders, etc. Other diseases that are relevant include chronic myelogenous leukemia; acute leukemia; cancer; T-cell leukemia; Alzheimer's disease; Parkinson's disease; multiple sclerosis; autoimmune disease; and surgery- and other trauma-induced immune dysfunction. 2-5A pathway defects are particularly manifested in diseases characterized by both chronic viral infection and immune cell defects.

Structural modification of the 2-5A molecule at the 3'-hydroxyl groups and elsewhere provides 2-5A analogues with remarkably increased metabolic stability to 2'-phosphodiesterases and cellular nucleuses, while maintaining the ability to activate RNase L and inhibit retroviral reverse transcriptase. Likewise modification of native 2-5A by substitution of the 3'-terminal nucleotide results in a more stable molecule. Persistent, high intracellular concentration of the metabolically stable 2-5A analogs are a consequence of their increased stability. Additionally, modifications of the 5'-terminal enhance the ability of the 2-5A molecule to activate RNase L.

The longer-lasting pharmacological activity of the 2-5A analogs offer a more favorable therapeutic ratio. This allows a decreased frequency of administration relative to 2-5A, which is metabolically unstable. Decreased frequency of administration is important due to the chronic nature of many afflictions characterized by 2-5A pathway defects. In addition, certain modifications of 2-5A derivatives that facilitate penetration into the cell further increase the therapeutic ratio by reducing the amount of the derivative that must be administered to give a therapeutic effect.

The 2-5A analogs are particularly useful in the treatment of infections caused by viruses. The 2-5A pathway defect associated with viral infection comprises the inactivation of the pathway caused by the virus' interference with the activation of 2-5A synthetase by dsRNA. In the absence of 2-5A synthetase activation, 2-5A production, and hence activation of RNase L, is reduced. According to the present invention, exogenous, metabolically stable 2-5A analog is administered to counteract this virally-caused defect in the 2-5A pathway. The 2-5A analogs, like authentic 2-5A, are capable of activating RNase L, which cleaves viral RNA.

The 2-5A analogs are particularly useful in protecting against infection by the various human T-cell leukemia viruses (collectively "HTLV"), such as HTLV-I, which cause cutaneous T-cell lymphoma; HTLV-II, which causes Sezary lymphoma: HTLV-III; and HTLV-IV, which is presently believed to be the etiologic agent of multiple sclerosis. Each of the HTLV viruses is a retrovirus. Also known as "HIV-1", HTLV-III is responsible for causing acquired immune deficiency syndrome ("AIDS"). The compounds are further believed useful in treating HIV-2, a second serologically distinct HIV subtype. Hereinafter (HIV) shall mean either HIV-1 or HIV-2, and any other HIV subtypes now or hereinafter known.

The 2-5A analogues are also particularly useful in protecting against infection by the various hepatitis viruses which cause viral hepatitis. The hepatitis B virus, in particular, is thought to be a composite of a retrovirus and a virus that employs replication of a DNA genome by DNA dependent DNA polymerase. The 2-5A analogues are also particularly useful in protecting against infection by viruses with a DNA genome, such as herpes viruses.

HTLV-infected patients, in particular HIV-1-infected patients, have been shown to demonstrate unusually low levels of 2-5A and/or RNase L activity in blood mononuclear cells. Blood mononuclear cells from healthy individuals, by contrast, display higher 2-5A levels, on average, and RNase L activity is readily detectable. Likewise blood mononuclear cells of chronic fatigue-inflicted individuals exhibit low 2-5A levels, and evidence the appearance of novel RNA cleavage products, distinct from the specific cleavage products observed in blood mononuclear cells from normal individuals.

While the practice of the invention is illustrated herein with regard to the treatment of HIV-1 infection, which is generally regarded as a prototypical retrovirus and HbV infection, which has properties both of retroviruses and DNA genome viruses, the method of the invention has application to the treatment of any diseases wherein the etiologic agent comprises a virus.

In addition, chronic virus infections are commonly associated with cytokine imbalances that produce additional pathogenic effects, including the accumulation of bioinactive 2-5A. The present invention operates to correct the effects of this imbalance by supplying bioactive 2-5A.

The preparation of certain of the compounds utilized in the practice of the present invention are described in U.S. Patents 4,859,768 and 4,924,624. The other compounds used in the practice of the invention may be prepared by following the general synthetic techniques described in those patents.

Examples of compounds for use in the method of the invention include the following core compounds, their corresponding 5' mono-, di-, and triphosphates, and the pharmaceutically acceptable salts of any of them:

### Mixed cordycepin/adenosine oligomers:

2',5'-C-C-C,
2',5'-A-A-C,
2',5'-A-C-C,
2',5'-C-C-A,
2',5'-C-A-C,
2',5'-C-C-A, and
2',5'-A-C-A, in addition to the various "tetramer" combinations of A and C, including but not limited to,
2',5'-C-C-C-C,
2',5'-A-A-A-C,
2',5'-A-A-C-C,
2',5'-A-A-C-A, and the like.

### Alkoxy compounds:

2',5'-A-A-A-3'-0-methyl
2',5'-A-A-A-3'-0-pentyl
2',5'-A-A-A-3'-0-hexyl
2',5'-A-A-A-3'-0-heptyl

### Amino compounds:

2',5'-A-A-A-3'-amino

### Miscellaneous compounds:

2',5'-A_{(Si)}A_{(Si)}A
2',5'-A-A-ara-A,
2',5'-A-A-Tu,
2',5'-Tu-Tu-Tu,
2',5'-I₃,
2',5'-xylo-A₃,
2',5'-xylo-A₄,
2',5'-C-C-dCF,
2',5-EHNA-A-A, and
5,6-dichlorobenzimidazylyl(2,'5')5,6-dichloro-3-benzimidazylyl(2',5')5,6-dichlorobenzimidazole riboside,
3'-deoxyadenylyl-(2'-5')-3'-deoxyadenylyl-(2'-4')-9-(4'hydroxybutyl)adenine, and
3'-deoxyadenylyl-(2'-5')-3'-deoxyadenylyl-(2'-4')-9-(4'hydroxyethoxy)adenine.

The two last mentioned compounds, characterized by an ether linkage between a cordycepin nucleotide and adenine, are from a group collectively referred to as "C-C-ether-A". Correspondingly, when the cordeycepin residues are substituted by adenosine, the compounds are referred to collectively as "A-A-ether-A".

### Inhibition of HBV Replication by 2-5A Derivatives

The antiviral activity of several 2-5A derivatives against hepatitis B virus was tested. Human liver cells chronically producing HBV (Acs et al., Proceedings of the National Academy of Sciences, USA 84:4641-4645 (1987)) were seeded into 24 well tissue culture plates and grown to confluence. 2-5A derivatives were then added at 20 µM for a continuous 9 day period. Culture medium was changed daily. Spent culture medium was analyzed for extracellular HBV DNA after 0, 3, 6 and 9 days. Treated cells were lysed 24 hours following the 9 day treatment and analyzed for intracellular genomic DNA forms. The protocol has been published by Korba and Milman (Antiviral Research 217:217 (1991)). The assay parameters are described in more detail below.

Both intracellular and extracellular HBV DNA were analyzed by procedures that are routine in the art in order to (1) allow for verification of compound efficacy, and (2) provide possible data on the target site in the HBV replication pathway for the compound from the examination of the pattern of viral replicative forms. The culture medium was changed daily during the treatment period to (1) prevent the buildup of potentially toxic metabolites from the test compounds, and (2) provide an analysis of HBV virion production during discrete 24-hour intervals which enables a quantitative comparison of any effect on virion production.

The analysis of HBV DNA was performed using blot hybridization techniques (Southern and slot blot) and [³²P]-labeled HBV-specific probes. HBV DNA levels were measure by comparison to known amounts of HBV DNA standards applied to every nitrocellulose membrane (gel or slot blot). An AMBIS beta scanner, which measures the radioactive decay of the hybridized probes directly from the nitrocellulose membranes, was used for the quantitative analysis. Standard curves, generated by multiple analyses, were used to correlate cpm measurements made by the beta scanner with relative levels of target DNA. The levels of HBV virion DNA released into the culture medium were analyzed by a slot blot hybridization procedure. HBV DNA levels were then compared to those at day 0 to determine the effect of drug treatment.

The levels of replicate intermediate and episomal monomers were used as an indicator of the relative levels of HBV replication. Integrated HBV DNA was used to normalize the relative amounts of DNA in each lane because the levels of this class of HBV DNA remain constant on a per cell basis. Inhibition of HBV DNA replication is indicated by the loss of replicative intermediates without changes in the level of integrated HBV DNA. In this assay, the following 2-5A derivatives showed anti-HBV activity: xyloA₃, A-A-ether-A, EHNA-AA, CAC, ACCA and dd benz.

To study these inhibitions further, 5 of those 2-5A derivatives were diluted and tested at 2 µM and 6 µM, in addition to 20 µM. The five 2-5A derivatives displayed marked anti-HBV inhibitory power at 20 µM and 3 of the 5 (A-A-ether-A, dd benz and CAC) were also very active at 2 µM. These results demonstrate that a variety of 2-5A derivatives are active against HBV. In accordance with the invention, it maybe concluded that anti-HBV of these and other 2-5A derivatives will be enhanced by adding 5' terminal modifications that augment the ability of the 2-5A derivatives to activate RNase L. Also in accordance with the invention, it may be concluded that anti-HBV activity will be augmented further by conjugating said 5' modified and 5' unmodified 2-5A derivatives with lipophyllic adducts such as cholesterol, palmitate, folate, etc.

To determine the therapeutic ratio of the 5 2-5A derivatives that were titrated for anti-HBV activity, their toxicity was measured with a conventional neutral red assay. The protocol for determining the toxicity of these compounds in culture is routine in the art and can be summarized as follows. 2.2.15 cells were grown to confluence in 96 well flat-bottom tissue culture plates and treated with compounds in 0.2 ml of culture medium per well. Four concentrations of each compound were assayed, each in triplicate cultures. Untreated control cultures were maintained on each 96 well plate. On each 96 well plate, wells containing no cells were used to correct for light scattering. Toxicity was determined by the inhibition of uptake of neutral red dye, as determined by the absorbance at 510 nanometers relative to untreated cells (Finter et al., J. Med. Chem 5:419 (1969)), 24 hours following day 9 of treatment.

No toxicity was observed for the aforesaid 2-5A derivatives at the highest concentration tested for antiviral activity (20 µM). Of the five 2-5A derivatives tested, four showed no toxicity at 9 times the effective antiviral dose (180 µM). The fifth, dd benz, showed low toxicity at 180 µM.

To study further the specificity of the HBV inhibition by the said 2-5A derivatives, their breakdown products were tested for possible anti-HBV activity. No anti-HBV activity was found with benzimidazole, dibenzimidazole₂, adenosine, cordecypin or 9-β-D-xylofuranosyladenine. These results show that the 2-5A derivatives specifically inhibit the replication of HBV at concentrations that are not toxic to uninfected cells and that the inhibition is due to the 2-5A derivatives and not to their metabolites.

### Viral Inhibition by Phosphorothioate Oligomers.

RNase L is a key functional enzyme of the 2-5A synthetase/RNase L antiviral defense pathway. The activation of this unique 2-5A-dependent endoribonuclease by 2-5A derivatives and the subsequent hydrolysis of viral RNA is critical in the inhibition of virus replication and regulation of cell growth. Some of the antiretroviral properties of interferon are mediated by activation of the 2-5A synthetase/RNase L pathway. Direct activation of RNase L by 2-5A derivatives bypasses the requirement for interferon in establishing an antiretroviral state. Modification of the 2-5A molecule at the 3'-hydroxyl groups and the 2',5'-internucleotide linkages has resulted in 2-5A derivatives that are metabolically stable (and therefore resistant to degradation by phosphodiesterases and phosphatases) and biologically active (capable of activating RNase L). Manipulation of synthetic procedures permits the design and synthesis of 2-5A derivatives which (i) display increased metabolic stability against nucleolytic degradation, (ii) are phosphatase- resistant, (iii) do not require 5'-phosphorylation for activation of RNase L, and (iv) can be transported into intact cells. Chirality was introduced into the 2',5'-phosphodiester bond of the 2-5A molecule as an approach to differentiate between RNase L activation and inhibition at the molecular level. The pure dimers and trimers of the diastereomeric phosphorothioate derivatives of 2-5A were first synthesized enzymatically. More recently, chemical synthesis, purification and identification of the two dimer, four trimer and eight tetramer diastereomers have been achieved via the phosphotriester and phosphoramidite approach. These chiral molecules exhibit striking biological activities which altered the dogma that three adenylate residues and two 5'-terminal phosphates were required to activate RNase L. In contrast to authentic 2-5A core molecules which are unable to activate RNase L, three of the four 2',5'-phosphorothioate trimer cores (RpRp, SpRp and RpSp) bind to and activate RNase L (at 10⁻⁵ M), as do the 2',5'-phosphorothioate 5'-monophosphates (pRpRp, pRpSp, and pSpRp) (at 10⁻⁸ M).

To determine whether these in vitro studies could be extended to an in vivo model with virus-infected cells, the 2',5'-phosphorothioate tetramer 5'--monophosphates were microinjected into the cytoplasm of HeLa cells and the cells were then challenged with VSV. The 2',5'-phosphorothioate tetramer 5'-monophosphates (pRpRpRp, pSpRpRp and pRpSpSp) all activated RNase L. However, the pSpSpSp isomer (at concentrations as high as 10⁻⁶ M) did not. Microinjection of the pSpSpSp inhibitor simultaneously with 2-5A eliminated protection against VSV replication. The Rₚ- and Sₚ-2',5'-phosphorothioates inhibit HIV-1 replication in intact cells in culture. The 2',5'-phosphorothioates protect target cells from HIV-1 infection by inhibition of HIV-1 RT (measured in Triton X-100-activated lysates of HIV-1 virions from H9/HTLV-IIIB culture supernatants). HIV-1 RT was not inhibited by A₃ or p₃A₃ at 0.25-256 µM. In contrast to 2-5A, the 2',5'-phosphorothioate tetramer 5'-monophosphate derivatives are very effective inhibitors of HIV-1 RT activity. The most effective inhibitor of HIV-1 RT in HIV-1-infected cell lysates is p₃A₃αS with 50% inhibition observed at 0.5 µM. AMPS did not inhibit HIV-1 RT up to 200 µM, suggesting that degradation products are not responsible for the inhibition observed with 2',5'-phosphorothioates.

d(T)₁₆ and tRNA^{Lys.3} bind specifically to the kinetically significant primer binding site of homogeneously pure recombinant p66 HIV-1 RT as demonstrated in UV crosslinking studies. Binding of oligo d(T)ₙ to p66 RT is not affected by dNTPs; in competition assays, the primer analogs, Sd(C)₂₈ and d(C)₁₉₋₂₄, the natural primer tRNA^{Lys.3} and p₃A₃αS inhibit oligo d(T)ₙ-p66/RT complex formation in a first-order exponential manner. The inhibition of HIV-1 RT by 2-5A and 2-5A derivatives occurs at the primer binding site. Under equilibrium binding conditions, 2',5'-p₃A₄ exhibits first order binding kinetics with one half competition at 55 µM (K_{D} = 31 x 10⁻⁶ M). 2',5'-p₃A₄αS exhibits first order binding kinetics with one half competition at 5.1 x 10⁻⁶ M (K_{D}=2-.9 x 10⁻⁶ M). 2',5'-A₃ core, 2',5'-pA₃, 3',5'-A₃ core, 3',5'-pA₃, 3',5'-p₃A₃ and ATP do not inhibit binding of HIV-1 RT to d(T)₁₆.

The 2',5'-cordycepin trimer core and 5'-monophosphate (1 µM) (when incorporated into antibody-targeted liposomes specific for the T cell receptor molecule CD3) inhibited 90% of HIV-1 replication. See Table 1 of Müller, W.E.G., Weiler, B.E., Charubala, R., Pfleiderer, W., Leserman, L., Sobol, R.W., Suhadolnik, R.J., and Schröder, H.C., Biochemistry 30, 2027 (1991). Dot-blot and gel-retardation assays showed that 2',5'-cordycepin trimer core and 5'-monophosphate interfere with the binding of tRNA^{Lys.3} to HIV-1 RT. See Figure 3 of Müller et al. pC₃ did not stimulate RNase L activity and displayed no effect on the amount of cellular RNA or protein. At a concentration of 10 µM, the cellular DNA polymerases α, β and γ were almost insensitive to C₃ or pC₃ [C = cordycepin].

We have available for the first time, antiretroviral molecules (2-5A derivatives) which possess dual effects on HIV-1-infected cells: (i) they activate the 2-5A synthetase/RNase L antiretroviral system and (ii) inhibit HIV-1 RT. With the 2',5'-phosphorothioate cores and their 5'-monophosphates, it is possible to prevent acute infection (by inhibition of HIV-1 RT) and simultaneously degrade HIV-1 mRNA by activation of RNase L.

### Conjugates of 2,5'-Oligoadenylate Derivatives Having Increased Cellular Uptake

To facilitate internalization of 2-5A derivatives into intact cells, bioactive 2-5A derivatives are conjugated to water-soluble vitamins (folic acid, biotin, riboflavin, or vitamin B₁₂) or to lipophilic chemical groups. By exploiting this technique, 2-5A derivatives may be delivered to intact cells without membrane damage. This approach involves (i) the synthesis of bioactive, metabolically stable derivatives, e.g., 2',5'-Cₙ, 2',5'-phosphorothioates (prepared according to U.S. Patent 4,924,624), and other metabolically stable derivatives such as those disclosed in U.S. Patent 4,-859,768. 2-5A derivatives according to the disclosure of the above-mentioned documents, have been designed, synthesized and tested for their antiretroviral effect at the level of RNase L and inhibition of RT. 2',5'-Oligonucleotides may be prepared enzymatically in multistep chemical syntheses via the phosphotriester and phosphoramidite approach, in milligram and gram amounts, respectively. The correct choice of appropriate blocking groups for sugar, base and phosphate protection determines yields, ease of purification and chromatographic behavior.

The successful facilitated delivery of biologically active, metabolically stable 2-5A derivatives is key to continuation of the mechanistic studies of inhibition of HIV-1 replication, and effective drug delivery. Several strategies for the assisted delivery of 2-5A derivatives into intact cells may be used.

### 2',5',-Oligoadenylate Lipophilic Conjugates

A first method of assisted delivery involves covalent conjugation of lipophilic groups (e.g., acyl or cholesteryl groups) to the 2',3'-terminus of the molecule. For example, the 2',5'-cordycepin trimer core may be synthesized and purified with palmitoyl (Formula III) or cholesterylformyl (Formula IV) ester linkages at the 2'-terminal hydroxyl group:

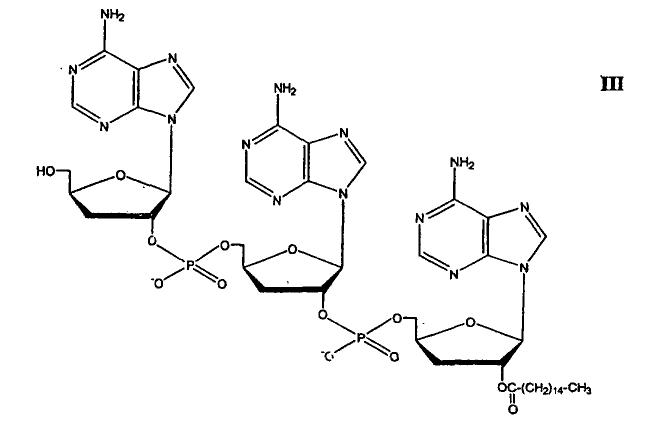

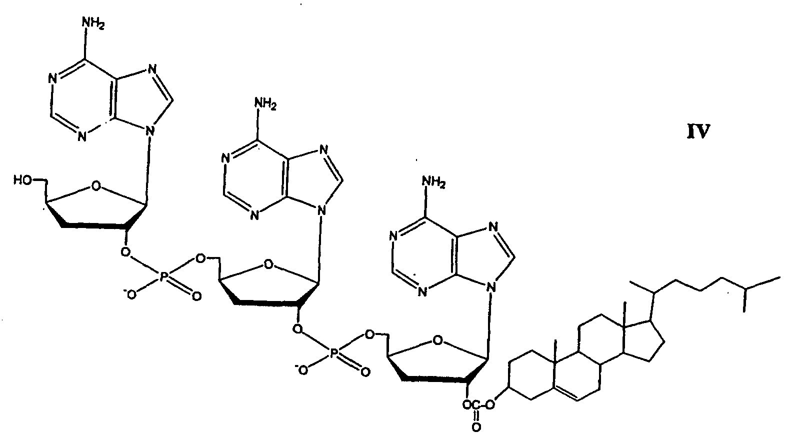

These esterified compounds have been found to inhibit HIV-1 replication in HIV-1 infected H9 cells in culture.

2-5A-analog cholesterol conjugates may be synthesized by reacting a conjugating compound of Formula V 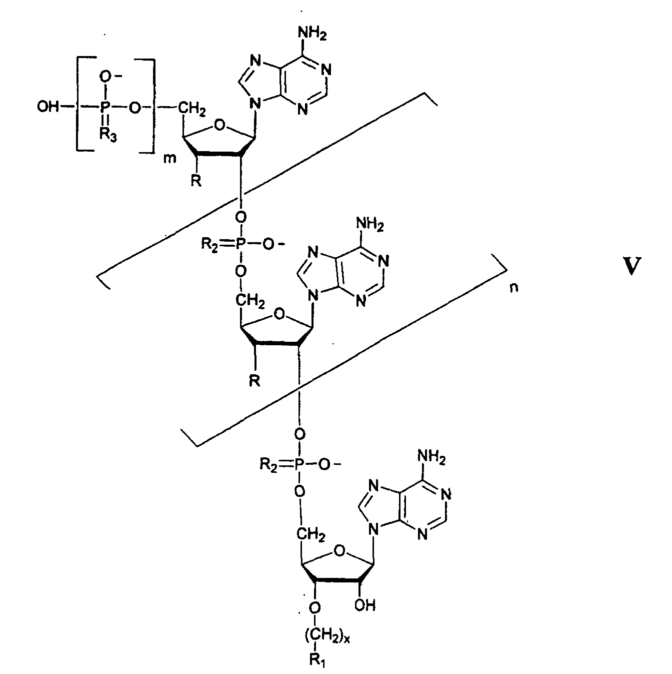 wherein
m is 0, 1, 2 or 3
n is an integer from 1 to 8,
R is independently selected from the group consisting of hydrogen and hydroxyl,
R₁ is COOH or NH₂, and
R₂ is independently sulfur or oxygen
R₃ is independently sulfur or oxygen, and
x is an integer from 1 to 17 with chlotesterylchloroformate in the presence of dimethylaminopyridine (DMAP) and dichloromethane. Purification is accomplished by column chromatography.

Preferably, not all R groups are hydroxyl, that is, at least one R is hydrogen. Preferably, n is 1 to 3, most preferably 1 or 2. The R₂ of the 5'-terminal phosphonyl group is preferably sulfur. Finally, the value of X is preferably from 1 to 8, most preferably 1 to 4. The conjugating 2-5A derivative according to Formula V may be prepared by functionalizing an appropriate 5'-phosphorylated 2',5'-oligoadenylate, 2',5'-oligocordycepin or mixed 2',5'-oligo(adenylate/cordycepin) oligomer, such as may be prepared from either U.S. Patent 4,464,359 or 4,859,768. The oligomer is functionalized by coupling the 2'-terminal nucleotide thereof to an alkyl linker -(CH₂)ₓ-R₁ wherein X and R₁ are defined above.

It should be appreciated that, in addition to oligomers containing phosphodiester internucleotide linkages, the conjugating 2-5A derivative of Formula V may be prepared by attaching the alkyl linker to the 2'-terminal nucleotide of a 2',5'-phosphorothioate oligonucleotide. The preparation of the 2',5'-phosphorothioates, including fully resolved enantiomers thereof (the phosphorothioates are optically active), is disclosed in U.S. Patent 4,924,624. It may be appreciated that by following the combined synthetic techniques of Patents 4,924,624 and 4,859,768, that a mixed 2',5'-(phosphodiester/phosphorothioate)-oligoadenylate may be prepared.

In the case where the R₂ group of all internucleotide bonds of the molecule comprise oxygen, i.e., the linkages comprise phosphodiester bonds, the 5'-monophosphates are readily prepared by reacting the corresponding unphosphorylated core compound with POCl₃. In the case wherein at least one internucleotide linkage comprises a phosphorothioate bond, i.e., R₂=sulfur, such treatment would result in the elimination of sulfur from the phosphorothioate internucleotide linkage, and the formation of a 2',5'-oligoadenylate. Thus, the 5'-monophosphates of phosphorothioate bond-containing core oligomers must be prepared from the corresponding fully protected core oligomer from which the monomethoxytrityl blocking groups on the 5'-terminal nucleotide has been removed. The procedure is described in detail in U.S. Patent 4,924,624, columns 26 through 31 thereof.

2-5-derivative/acyl conjugates may be synthesized by reacting the conjugating compound with acyl chloride, according to standard techniques. Alternatively, the 2',5'-oligomer may be synthesized directly with an acyl group or cholesteryl formyl group esterified to the 2'-terminal hydroxyl group. One such method is illustrated by but not limited to the preparation of the cholesterol conjugated and acylated cordycepin trimer compounds comprising compounds **12** and **13** of Scheme 1.

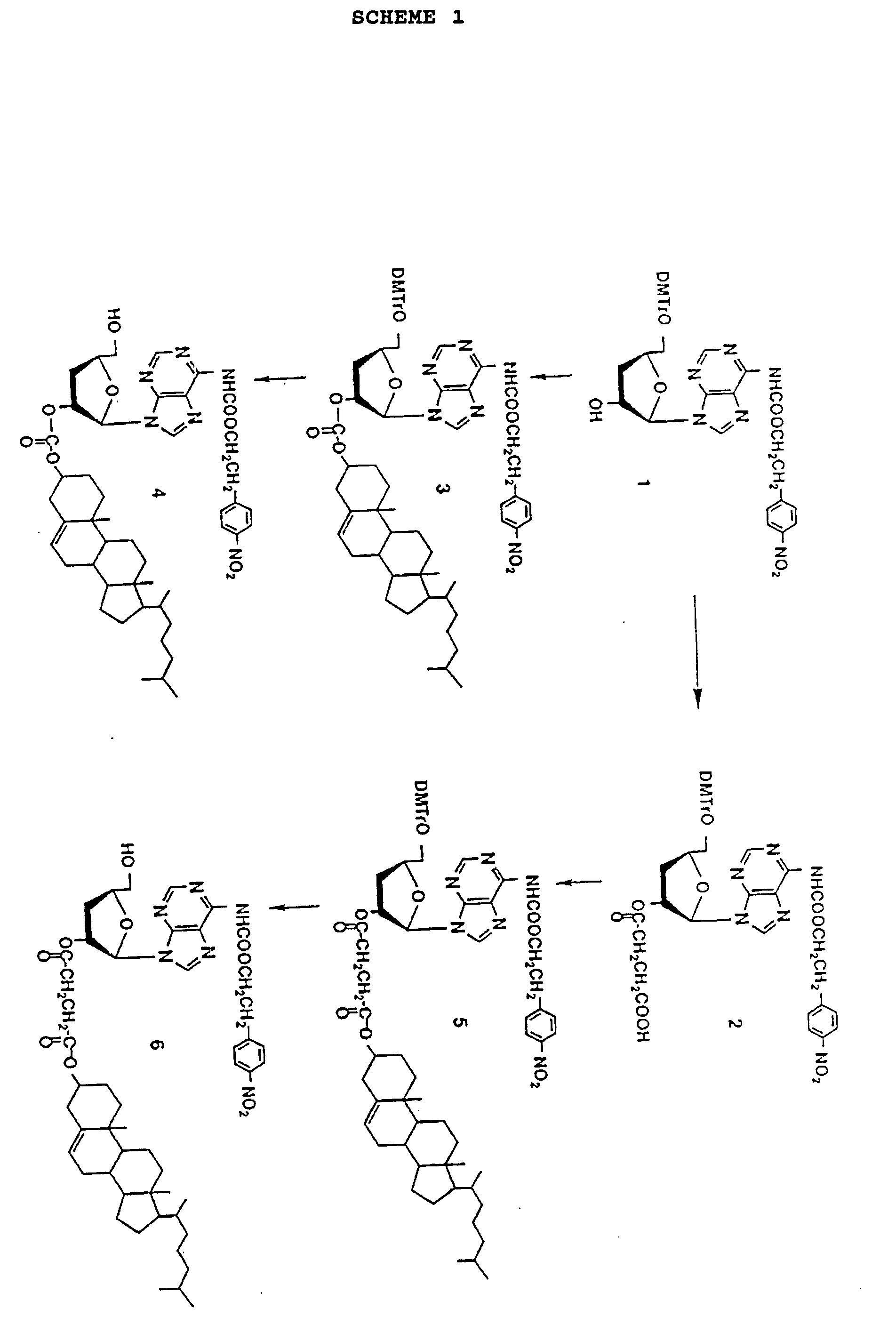

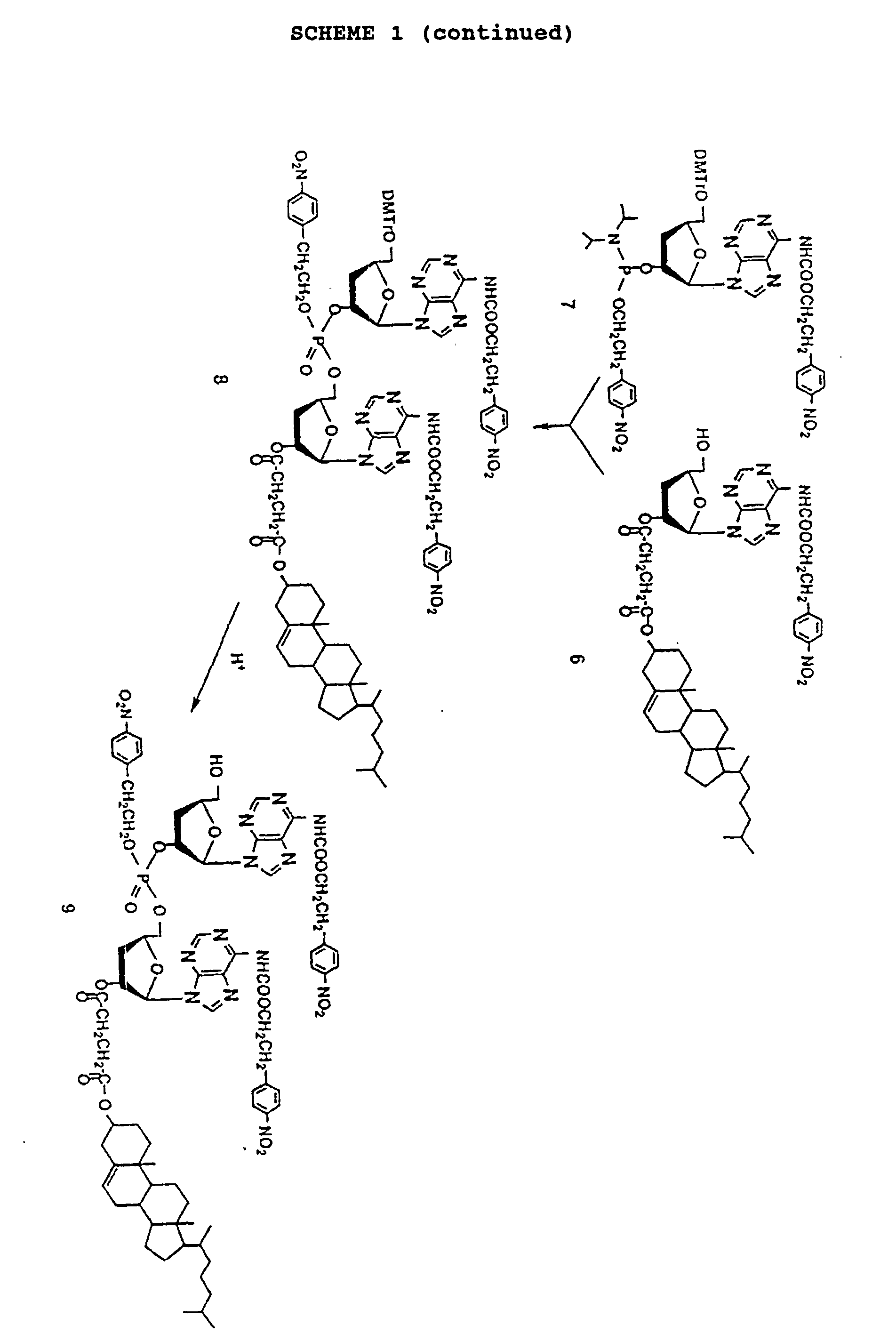

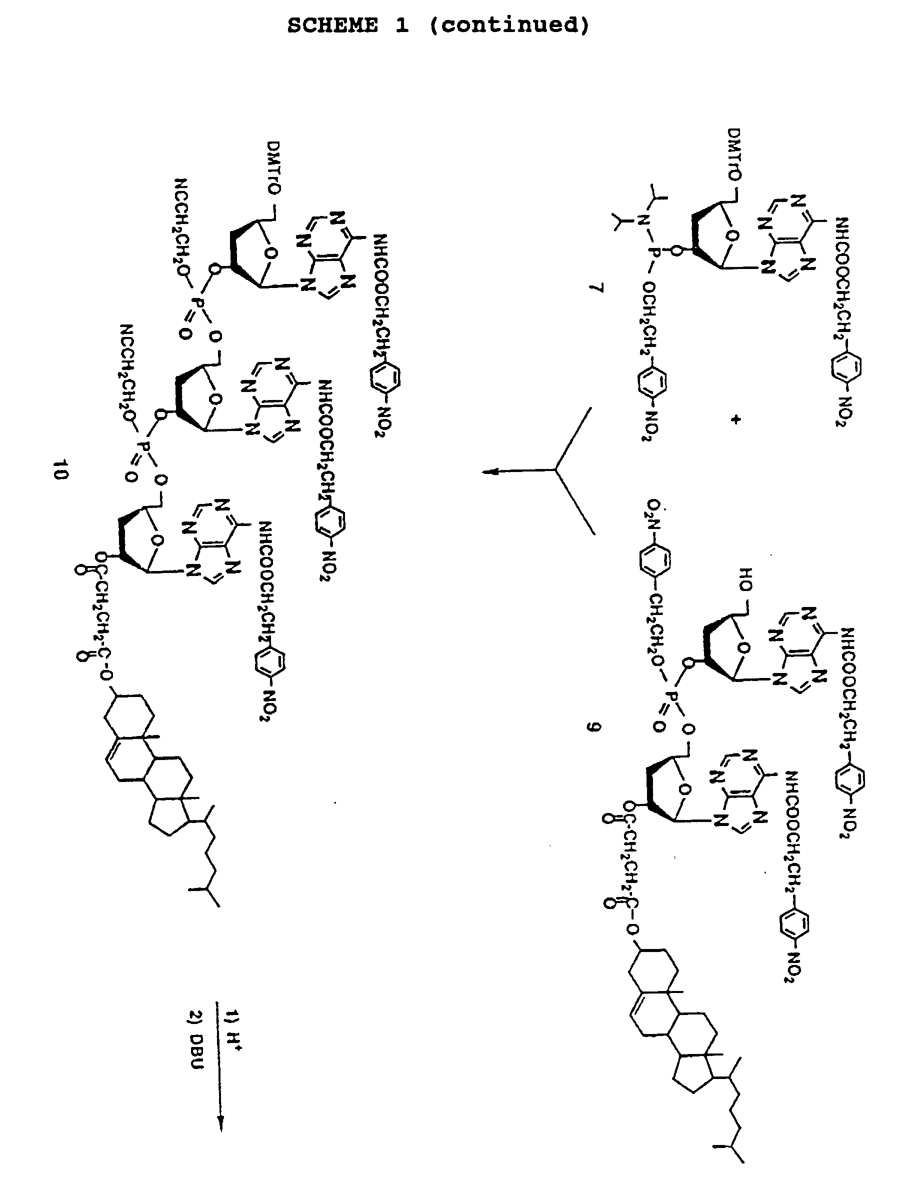

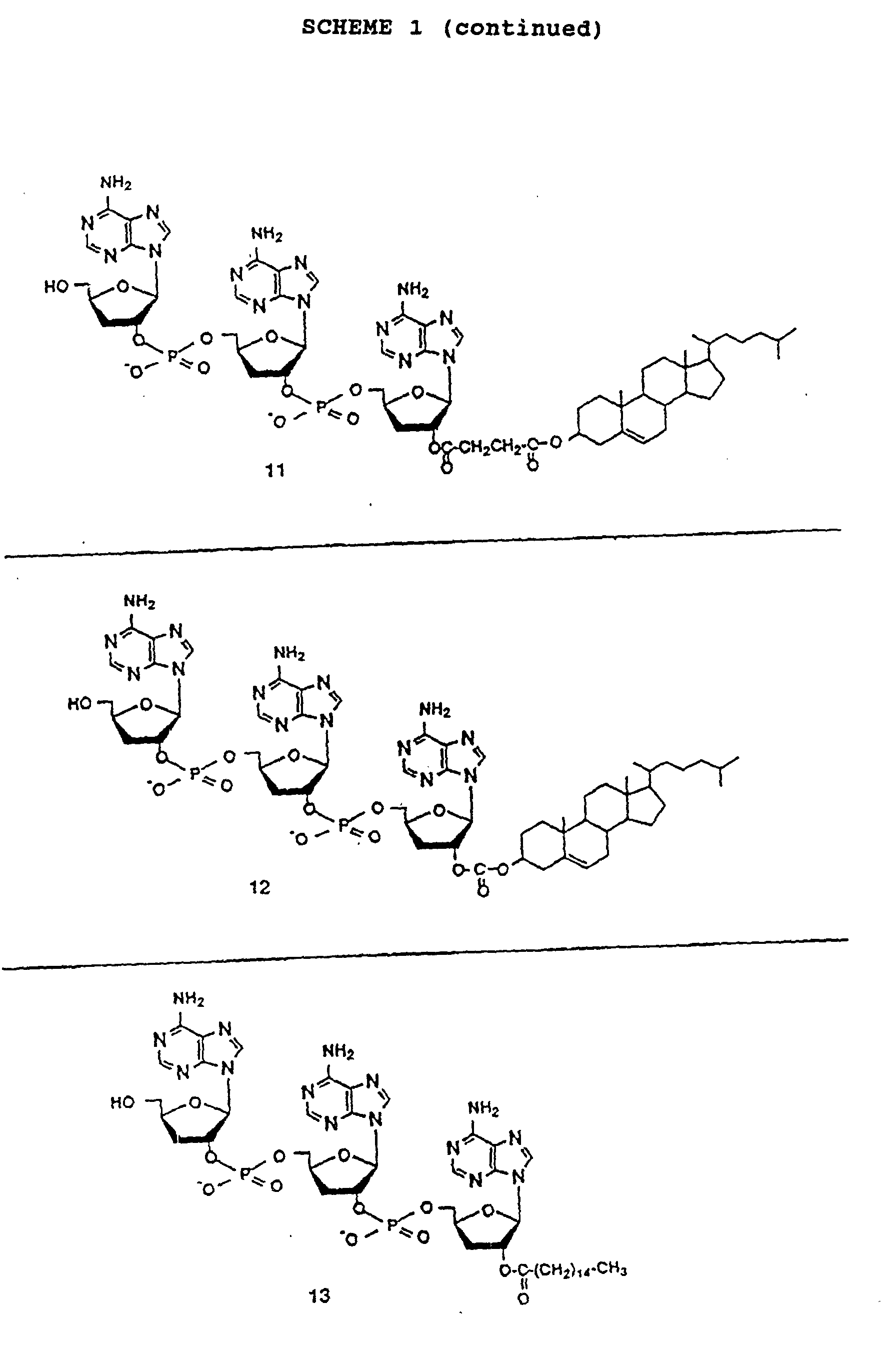

According to Scheme 1, a multistep chemical synthesis starts from 5'-O-dimethoxytrityl-N⁶-p-nitro-phenylethoxycarbonyl-cordycepin (**1**) and the corresponding 2'-O-succinate derivative (**2**). Compound **1** is reacted with colesteryl chloroformate to the carbonate **3** which is used as the 2'-terminal building block after detritylation to **4**. The succinate **2** is condensed with cholesterol to the ester **5**, and detritylation leads to compound **6**.

The synthetic route to the trimer conjugates is illustrated in Scheme 2 by one example starting from compound **6**. This cordycepin derivative is first reacted with the fully protected phosphoramidite **7** to give in excellent yield with the dimer **8**. Detritylation at the 5'- end forms **9** and subsequent condensation with a second molecule of **7** gives the fully protected trimer **10**. The latter is sequentially treated with acid to cleave the dimethoxytrityl group and by DBU to cleave the p-nitrophenylethoxycarbonyl groups simultaneously, to afford the free cordycepin trimer conjugate **11**. The advantage of this approach can be seen from the fact that the ester function between cholesterol and the succinate spacer does survive all the chemical manipulations.

In an entirely analogous manner the cholesterylcarbonate **4** is reacted according to the same sequence of steps to prepare the cordycepine trimer conjugate **12**.

Also in an entirely analogous manner, the cordycepin trimer conjugate **13**, which carries an acyl residue at the 2'-terminal end of the oligonucleotide, is prepared.

Lipophilic conjugates according to the present invention have the potential to enter the cell by diffusion, owing to their lipophilic nature. They may be introduced into intact cells with substantially the same biological activity as authentic 2-5A. Once inside the cell, the conjugates are hydrolyzed and release the 2-5A derivative.

### 2',5'-Oligoadenylate Vitamin conjugates

A second method of assisted oligonucleotide delivery involves covalent conjugation of 2-5A derivatives to water-soluble vitamins such as biotin, folic acid, riboflavin or vitamin B₁₂. Covalent conjugation of 2-5A derivatives to water-soluble vitamins provides a method for co-delivery of active molecules into intact cells by exploiting receptor-mediated endocytosis with retention of essentially full biological activity.

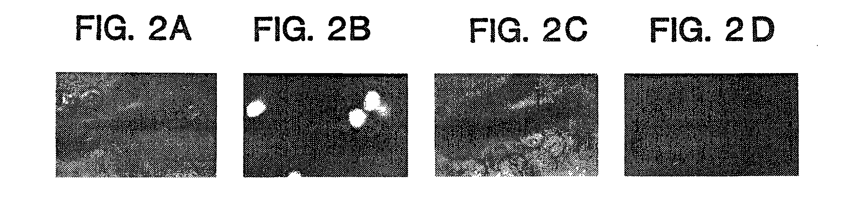

As a mode for demonstrating this principle, we have undertaken internalization of fluorescein-bovine serum albumin (BSA) covalently conjugated to folate in H9 cells in culture (Figure 2A and 2B). In control experiments, H9 cells incubated for 5 hours with fluorescein-BSA, but not conjugated to folate, were not taken up (Figures 2C and 2D). These results demonstrate the presence of folate receptors on H9 cells, which receptors may be used for targeted delivery of 2-5A derivatives. The folate receptors are able to bind to the folate-conjugated molecule and undergo uptake into 80% of the cells in 5 hours.

2-5A derivatives can be covalently conjugated to water-soluble vitamins to facilitate delivery of the polar 2-5A molecule into intact cells without membrane damage. The water-soluble vitamins are covalently conjugated to the 2',3'-terminus of the 2-5A molecule to exploit receptor-mediated endocytosis for uptake into intact cells. Nondestructive delivery of polar molecules covalently conjugated to biotin, folic acid, riboflavin and vitamin B₁₂ into the cytoplasm and subsequent efficient uptake via receptor-mediated endocytosis has been well demonstrated. In this way, membrane damage arising from other established methods (such as microinjection, electroporation, sonication, detergent permeabilization) can be avoided by employing the natural vitamin endocytosis pathway to accomplish uptake. Vitamin uptake occurs in all dividing cells at reasonable rates. Based on the known efficiency of uptake of vitamin conjugates, it is believed that an intracellular concentration of a 2-5A/folic acid conjugate of about 10⁻⁶ M can be achieved; these concentrations are sufficient to activate RNase L and inhibit HIV-1 RT. Similar intracellular concentrations are expected with 2-5A/biotin and the 2-5A/vitamin B₁₂ conjugates on the basis of the similar numbers of folate, biotin and vitamin B₁₂ receptors.

### 2-5A Vitamin B₁₂ Conjugate

A vitamin B₁₂ covalent conjugation of 2-5A or a bioactive 2-5A derivative is prepared by first converting vitamin B₁₂ to the mono-acid derivative thereof by mild acid hydrolysis (0.4 M HCl, 72 hours at room temperature). A 2-5A conjugating compound, such as a compound according to Formula V (R₁ = NH₂), is reacted with the carboxyl group of vitamin B₁₂ using an appropriate coupling agent such as 1-ethyl-3-(dimethyl-amino-propyl)-carbodiimide HCl (EDAC). The 2-5A/vitamin B₁₂ conjugate is purified by either chromatography on Sephadex G-25 or reverse phase HPLC. Characterization is based on the reported molar extinction coefficients for 2-5A and vitamin B₁₂.

### 2-5A/Folic Acid Conjugate

The 2-5A/folic acid conjugate is synthesized by reacting a conjugating compound, such as a compound according to Formula V (R₁ = NH₂), with a suitable coupling reagent such as EDAC, followed by purification using either column chromatography, thin layer chromatography or HPLC.

### 2-5A/Biotin Conjugate

Biotinylated 2-5A conjugate may be synthesized by reacting commercially available N-hydroxysuccidimidyl biotin with a Formula V compound (R₁ = NH₂), followed by purification as described for the 2-5A/folic acid conjugate.

### Preparation of 2',5'-Cordycepin Analogs Containing 3'-Terminal Acyclic Nucleoside

Trimer "core" cordycepin analogs according to Formula I, i.e., compounds wherein m is O and n is 1 may be prepared according to Scheme 2, wherein "bz" is benzoyl, "MeOTr" is monomethoxytrityl, "npe" is 2-(4-nitrophenyl)ethyl and "npeoc" is [2-(4-nitrophenyl)-ethoxy]carbonyl. For illustration purposes, the compounds are prepared as ammonium salts, it being understood that the invention is not so limited. The Scheme is exemplified by the preparation of four compounds, 18, 19, 20 and 21.

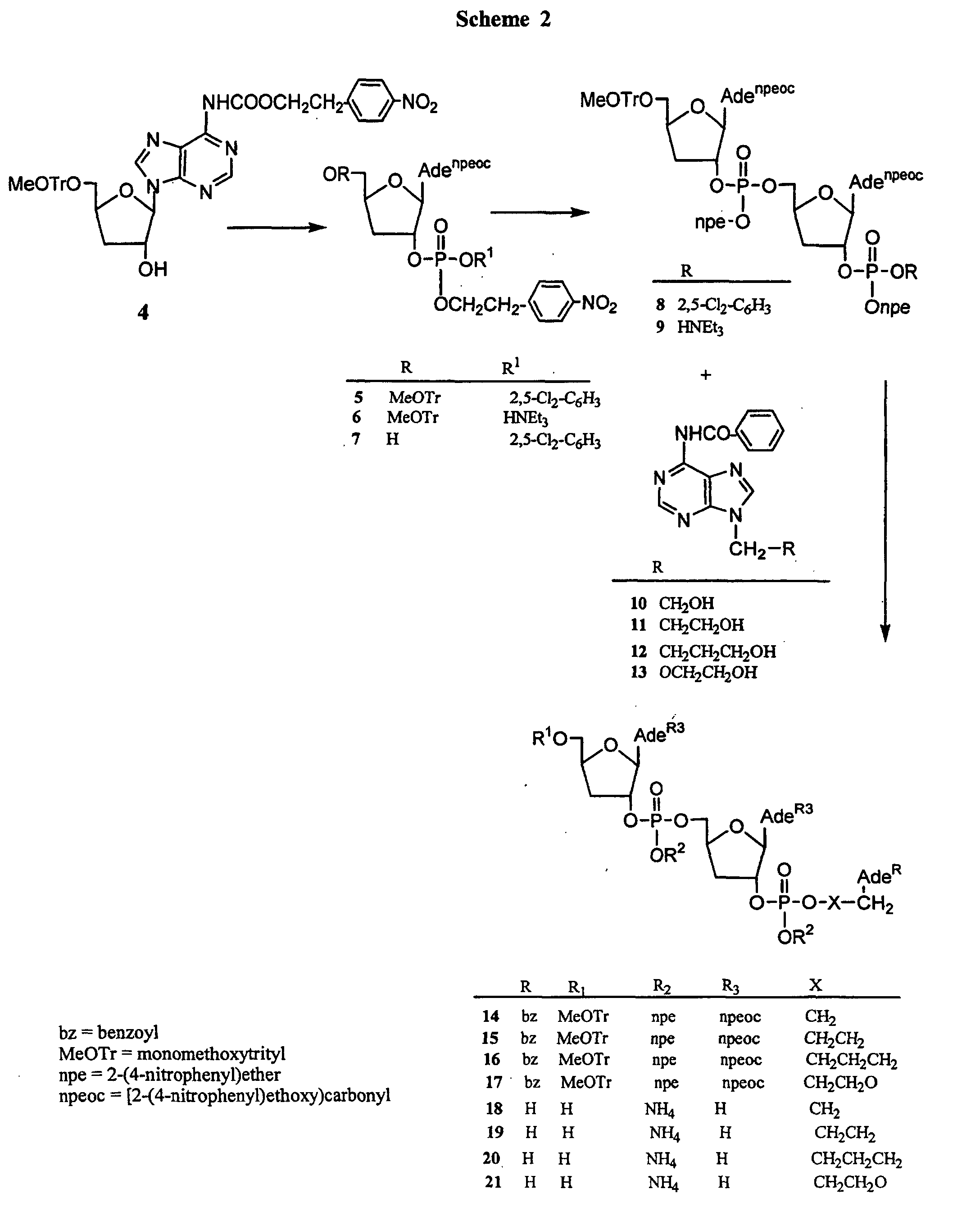

The dinucleotide monophosphodiester **9** was prepared from 3'-deoxy-5'-O-(monomethoxytrityl)-N⁶-[2-(4-nitrophenyl)ethoxycarbonyl]adenosine **(4)** according to the method of Charubala et al., Helv. Chim. Acta 70, 2028 (1987). Compound **4** which may be prepared according to Charubala et al., was taken and converted into the 2'-phosphotriester **5** with a 2,5-dichlorophenyl and a 2-(4-nitrophenyl)ethyl group at the phosphate function (Scheme 1) according to the method of said Charubala et al. reference. From **5**, the 2,5-dichlorophenyl group was cleaved off with 4-nitrobenzaldehyde oxime to give diester **6**. On the other hand, the 5'-**O**-monomethoxytrityl group was removed by acid to give the 5'-hydroxytriester **7**. These two building blocks **6** and **7** were condensed in presence of 2,4,6-triisopropylbenzenesulfonyl chloride and 1-methyl-1**H**-imidazole to yield in 88%, after purification and drying, the dimeric phosphotriester **8**. Again, the 2,5-dichlorophenyl group from this dimer block was cleaved off by the oximate method to give the corresponding dinucleotide monophosphodiester **9**.

For the syntheses of trimers **14-17,** the above dimeric phosphodiester **9** was condensed with the suitably blocked appropriate acyclic nucleosides **10-13** in presence of 2,4,6-triisopropylbenzenesulfonyl chloride and l-methyl-lH-imidazole in yields of 70-75%. Acyclic nucleosides **10**-**13** may be prepared according to the procedure of Tychinskaya et al., Bioorg. Khim (USSP) 5, 1059 (1979) and Mikhailov et al., Izv. Akad. Nauk. Ser. Khim. 2582 (1974). Dimer diester **9** (1mmol) and the appropriate acyclic nucleoside analog **10-13** (0.75 mmol) were coevaporated with dry pyridine (5 x 10 ml), and finally dissolved in pyridine (10 ml). Then 0.46 ml (6 mmol) of l-methyl-lH-imidazole and 0.606 g (2 mmol) of 2,4,6-triisopropylbenzenesulfonyl chloride were added successively. The mixture was stirred at room temperatures for 20 hours, then diluted with CHCl₃ (100 ml), and washed with H₂O(2 x 50 ml). The organic phase was dried and evaporated, and after coevaporation with toluene (3 x 20 ml), the residue was purified by CC (silica gel, 10 x 2.5 cm, CHCl₃/MeOH 100:1) : **14-17** as colorless foams in 85-90% yield. Accordingly, to a solution of protected trimer **15-18** (0.028 g; 15 µmol) was added 0.5M DBU in dry pyridine (5 ml). The mixture was stirred at room temperature for 20 hours, neutralized with 1M AcOH in pyridine (2.5 ml), and evaporated. The residue was stirred with concentrated NH₃ (15 ml) and after stirring for 48 hours, the solvent was removed *in vacuo* and the residue detritylated by treatment with 80% AcOH/H₂O (4 ml) at room temperature for 24 hours. After evaporation, the residue was coevaporated several times with H₂O, taken up in H₂O and applied onto a DEAE A-25 Sephadex® column (60 x 1 cm) and eluted with a linear gradient 0.001-0.3M (Et₃NH) and HCO₃ (TBK) buffer (pH 7.5). The product fractions were evaporated and coevaporated several times with H₂O and further purified by paper chromatography (i-PrOH/conc. NH₃/H₂O 6:1:3). The product band was cut out and eluted with H₂O and lyophilized to give3'-deoxyadenylyl-(2'-5')-3'-deoxyadenylyl-(2'-2')-9-(2'-hydroxyethyl)adenine (diammonium salt; **18**), 3'-deoxyadenylyl-(2'-5')-3'-deoxyadenylyl-(2'-3')-9-(3'-hydroxypropyl)adenine (diammonium salt; **19**), 3'-deoxyadenylyl-(2'-5')-3'-deoxyadenylyl-(2'-4')-9-(4'-hydroxybutyl)adenine (diammonium salt; **20**), and 3'-deoxyadenylyl-(2'-5')-3-deoxyadenylyl-(2'-2")-9-[(2"-hydroxyethoxy)methyl]adenine diammonium salt; **21**, respectively, as colorless powders in 75-85% yield. The purity of all products was checked by TLC, UV, and ¹H-NMR spectra.

All trimers were deblocked sequentially, using 0.5M DBU (1,8-diazabicyclo[5.4.0]undec-7-ene) in dry pyridine for 20 hours at room temperature to cleave the 2-(4-nitrophenyl)ethyl groups, NH₃ in dioxane to remove the benzoyl groups, and finally acid treatment to deblock the 5'-**O**-monomethoxytrityl group yielding the corresponding fully deblocked trimers **18-21**, which were purified by DEAE sephadex column chromatography and paper chromatography. The trimers were characterized by TLC, UV, HPLC, and ¹H-NMR spectra (data not shown).

Tetramers (C-C-C-ether-A) may be prepared by substituting for the dinucleotide monophosphodiester **9** in Scheme 2 the corresponding protected trinucleotide **9a** which is condensed with the suitably blocked appropriate acyclic nucleoside, as above. Similarly, it should be clear that higher oligomers of the present 3'-acyclic cordycepin oligomers may be prepared by selecting the appropriate higher oligonucleotide for condensation with the acyclic nucleoside.

The 5'-monophosphates of the unphosphorylated core molecules may be prepared from the fully blocked trimers, e.g., **14**-**17** by detritylation with acid treatment, followed by reaction with di-p-nitrophenylethylphosphoryl chloride. Further deblocking and chromatography results in isolation of the 5'-monophosphate oligomers. The 5'-monophosphorylation procedure may be carried out according to the method of Example 6 of U.S. Patent 4,859,768.

The 5'-diphosphates and 5'-triphosphates of the core molecules may be prepared according to the procedure of the aforesaid patent. Briefly, they may be prepared by adding 0.5 mM of tributylammonium pyrophosphate dissolved in 5 ml of dimethylformamide to 0.1mM of monophosphorylated core as the anhydrous tributylammonium salt in 1 ml of dimethylformamide and 0.5 mM of 1,1'-carbonyldiimidazole. After 20 hours at room temperature, the reactants are treated with 5ml of methanol, evaporated to dryness and chromatographed on a 2x20 cm DEAE cellulose column. The 5'- di- and triphosphates are isolated following a linear gradient (0-0.4M in 3 1 at pH 7.5) of triethylammoniumbicarbonate, according to the method of Hoard, et al., J. Amer. Chem. Soc. 87, 1785-1788 (1965). The 5'-diphosphates and 5'-triphosphates may then be purified by DEAE-Sephadex® A25 and Sephadex®.

The 2-5A analogs and conjugates may be administered according to the methods described in Patents 4,924,624, 4,859,768 and International Patent Application No. PCT/US85/03634 (International Publication No. WO 89/03683) in the dosages recommended therein. Thus, for pharmaceutical use, the 2',5'-oligoadenylate analogs may be taken up in pharmaceutically acceptable carriers. Such carriers for preparation of pharmaceutical compositions may be either organic or inorganic, solid or liquid in nature. Suitable liquid carriers include water and alcohols such as ethanol, benzyl alcohol and poly(ethylene glycols). The preferred liquid carriers for injectable preparations include water, saline solution, dextrose solution and glycol solutions such as aqueous propylene glycol or aqueous poly(ethylene glycol). The properties of the formulations may be enhanced by the addition of one or more adjuvants possessing properties as viscosity enhancers, surfactants, pH modifiers, preservatives, sweeteners, stability enhancers, coloring agents, suspending agents, granulating agents, coating agents, disintegration modifiers, propellants, emulsifying agents and hymectants. The resulting compositions may be in the form of solutions, suspensions, tablets, capsules, ointments, elixirs, injectable compositions and the like.

The dosage administered depends upon the severity of the infection or affliction and the size and weight of the subject. The dosage may vary over a wide range depending on the nature of the affliction, and the size and weight of the subject. According to one embodiment, the compounds are prepared as a solution of 0.1-100 mg/ml in water, phosphate-buffered saline or other appropriate fluid, or may be prepared as a tablet containing 0.01-1 gram active compound.

The compounds may be administered in the form of water-soluble salts. Pharmaceutically acceptable water-soluble salts include, for example, the sodium, potassium and ammonium salts of the active compounds. They are readily dissolved in water or saline solution. the formulation may contain additional agents, such as a sugar or protein, to maintain the osmotic balance.

The 2',5'-oligoadenylate analogs may be administered in doses of about 0.1 mg to about 1 gram to animals or humans afflicted by, or suspected of affliction by, or at risk or affliction by, any of the various conditions characterized by a 2-5A pathway defect. The total daily dosage may vary, for example, from about 0.001 gram to about 1 gram, although lower or higher amounts may be administered. A preferred daily dose is from about 0.01 gram to about 0.1 g of active ingredient. The compounds may be administered by any of several routes, including, but not limited to, intravenous injection, intraperitoneal or intramuscular injection, and oral administration. Techniques for accomplishing such administration are routine and known in the medical art. Administration may be as frequent as several time a day or as infrequent as weekly. For intravenous injection, particularly for treatment of HIV, a solution containing about 0.1 to about 1.0 milligram per ml of active ingredient is preferred.

It is also contemplated that the 2',5'-oligoadenylate analogs may be administered topically to treat skin lesions associated with any of the disease states characterized 2-5A pathway defect. A sufficient amount of a preparation containing one or more of the 2',5'-oligoadenylate analogs may be applied to cover the lesion or affected area. An effective concentration of active agent is from about 10⁻³ M to about 10⁻⁵ M, with about 10⁻⁴ M being preferred.

For conjugates if 2-5A analog and cell penetration-enhancing adducts, the dosage is based upon the weight of the 2-5A analog moiety. In addition to administration with conventional carriers, the 2-5A analogs may be administered by a variety of specialized oligonucleotide or nucleic acid delivery techniques, such as by encapsulation in unilameller liposomes or reconstituted sendai virus envelopes, or by conjugation to carrier molecules such as poly(L-lysine). Such methods are disclosed in International Patent Application No. PCT/-US85/03634.

In addition, 2-5A derivatives and conjugates may be administered via microscopic nanometer-size polystyrene carriers which dissolve in the bloodstream to release the active drug. Other suitable carriers are discussed in the aforementioned patent documents.

## Claims

1. A compound according to the formula 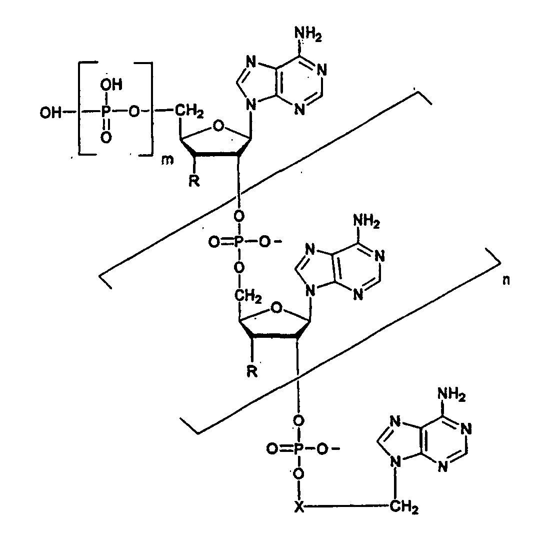 wherein
n is a whole positive integer from 1 to 8,
m is 0, 1, 2 or 3,
R is hydrogen,
X is selected from C₁ to C₆ alkylene and C₁ to C₆ alkyleneoxy, or
a pharmaceutically acceptable salt thereof.

2. A compound according to claim 1 wherein x is selected from C₁ to C₆ alkylene and C₁ to C₆ alkyleneoxy.

3. A compound according to claim 1 wherein the compound is selected from 3'-deoxyadenylyl-(2'-5')-3'-deoxyadenylyl(2'-2')-9-(2'-hydroxyethyl)adenine, the 5' mono-, di- and tri-phosphates thereof, and any pharmaceutically acceptable salts of any of them.

4. A compound according to claim 1 wherein the compound is selected from 3'-deoxyadenylyl-(2',5')-3'-deoxyadenylyl-(2',3')-9-(3'-hydroxypropyl)adenine, the 5' mono-, di- and tri-phosphates thereof, and any pharmaceutically acceptable salts of any of them.

5. A compound according to claim 1 wherein the compound is selected from 3'-deoxyadenylyl-(2',5')-3'-deoxyadenylyl-(2',4')-9-(4'-hydroxybutyl)adenine, the 5' mono-, di- and tri-phosphates thereof, and any pharmaceutically acceptable salts of any of them.

6. A compound according to claim 1 wherein the compound is selected from 3'-deoxyadenylyl-(2',5')-3'-deoxyadenylyl-(2',2')-9-[(2"-hydroxethoxy)methyl]adenine, the 5' mono-, di- and triphosphates thereof, and any pharmaceutically acceptable salts of any of them.

7. A pharmaceutical composition comprising a compound according to any of claims 2 to 6 and a pharmaceutical carrier.

8. A compound according to any of claims 1 to 6 for use in medicine.

9. Use of a compound according to any of claims 1 to 6 in the manufacture of a medicament for inhibiting viral infection in a mammal.

10. A conjugate comprising a compound according to the formula 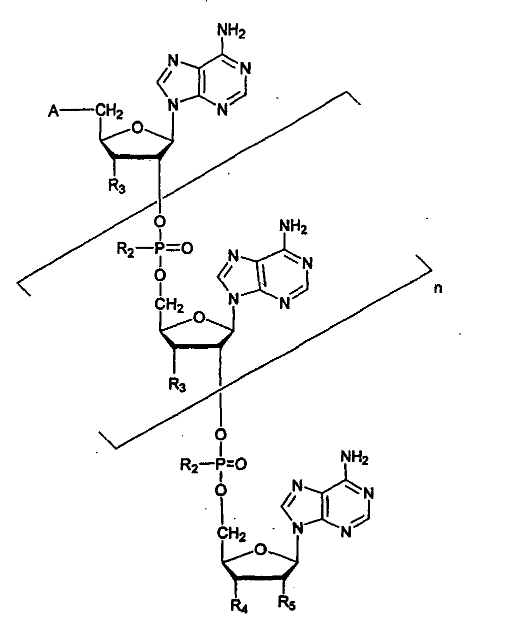 wherein
A is 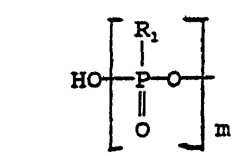 or 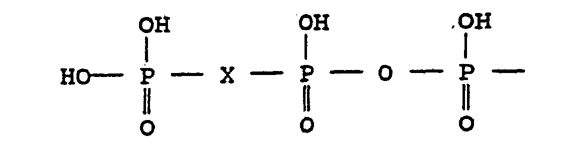 X is NH or CH₂;
m is zero, 1 2 or 3;
n is an integer from 1 to 8;
each R₁ is independently selected from O⁻, S⁻, sulfate, Se⁻, C₁ to C₈ alkyl and C₁ to C₈ alkoxy;
each R₂ is independently selected from O⁻, S⁻, sulfate, Se⁻, C₁ to C₈ alkyl and C₁ to C₈ alkoxy;
each R₃ is independently selected from hydrogen, hydroxyl, amino and -OSi(CH₃)₂C(CH₃)₃;
R₄ and R₅ are independently selected from hydrogen; hydroxyl; amino; C₁ to C₈ alkyl ; C₁ to C₈ alkoxy; C₁ to C₈ alkylamino, alkylcarbonyl, alkylcarboxyl and haloalkyl; and C₁ to C₈ alkoxyamino, alkoxycarbonyl, alkoxycarboxyl and haloalkoxy; or pharmaceutically acceptable salts thereof,
excluding authentic 2',5'-oligoadenylate and salts thereof,
said compound being covalently linked to an adduct through a hydroxyl oxygen at the 2'- or 3'-position of the 2'-terminal nucleotide of the compound, which adduct is selected from (i) vitamins which have corresponding cell surface receptors for receptor-mediated endocytosis, (ii) adducts containing the cholesteryl groups, and (iii) acyl groups of the formula 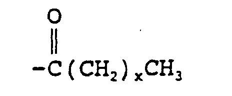 wherein x is an integer from 1 to 20, and which adduct results in enhanced penetration of the compound into intact cells.

11. A conjugate according to claim 10, wherein the adduct is vitamin B₁₂.

12. A conjugate according to claim 10, wherein the adduct is biotin.

13. A conjugate according to claim 10, wherein the adduct is folic acid.

14. A conjugate according to claim 10, wherein the adduct is lipophilic.

15. A conjugate according to claim 10, wherein
A is 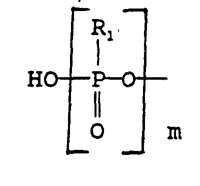 m is zero, 1, 2 or 3,
n is an integer from 1 to 8,
each R₃ and R₄ is independently selected from hydrogen and hydroxyl,
each R₂ is independently selected from S⁻ and O⁻,
R₅ is hydroxy,
or a pharmaceutically acceptable salt thereof, which compound is covalently linked through the 2'-position of the 2'-terminal nucleotide thereof to said adduct.

16. A conjugate according to claim 15, wherein the adduct is a vitamin.

17. A conjugate according to claim 16, wherein the vitamin is selected from the group consisting of B₁₂, folic acid and biotin.

18. A conjugate according to claim 15, wherein the adduct is lipophilic.

19. A conjugate according to claim 15, wherein the adduct contains a cholesteryl group.

20. A conjugate according to claim 18 wherein the adduct is an acyl group of the formula 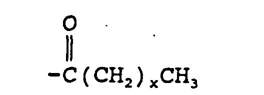 wherein X is an integer from 1 to 20.

21. A conjugate according to claim 20 wherein the adduct is palmitoyl.

22. A pharmaceutical composition comprising a compound according to the formula 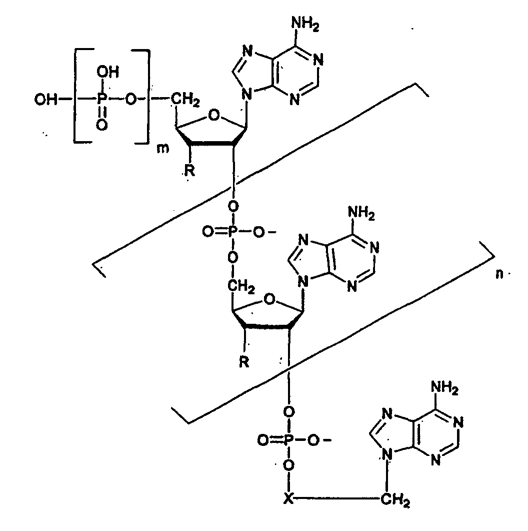 wherein
n is a whole positive integer from 1 to 8,
m is 0, 1, 2 or 3,
R is independently hydrogen or hydroxyl,
X is selected from C₁ to C₆ alkylene and C₁ to C₆ alkyleneoxy, or
a pharmaceutically acceptable salt thereof, and a pharmaceutical carrier.

23. A composition according to claim 22, wherein X is selected from C₁ to C₃ alkylene and C₁ to C₃ alkyleneoxy and n is 1 to 3.

24. A composition according to claim 23, wherein X is ethyleneoxy.

25. A composition according to any of claims 22 to 24, wherein R is hydrogen.

26. A composition according to any of claims 22 to 24, wherein R is hydroxyl.

27. A composition according to any of claims 22 to 26 wherein m is zero.

28. A compound according to the formula 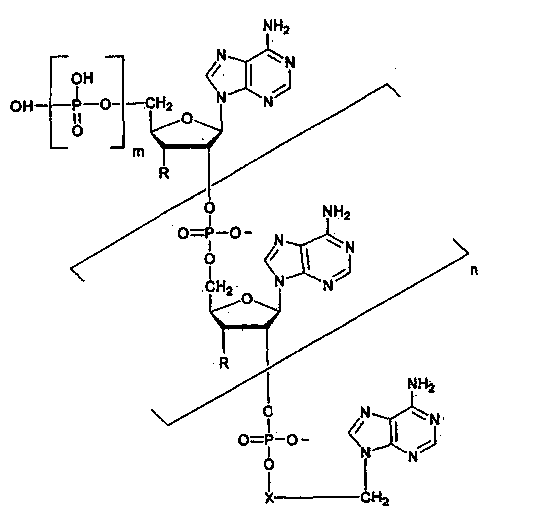 wherein
n is a whole positive integer from 1 to 8,
m is 0, 1, 2 or 3,
R is hydroxyl,
X is selected from C₁ to C ₆ alkylene and C ₁ to C ₆ alkyleneoxy, for use in medicine.

29. A compound according to claim 28, wherein X is selected from C₁ to C₃ alkylene and C₁ to C₃ alkyleneoxy and n is 1 to 3.

30. A compound according to claim 29, wherein X is ethyleneoxy.

31. A compound according to any of claims 28 to 30, where m is zero.

32. Use of a compound according to the formula 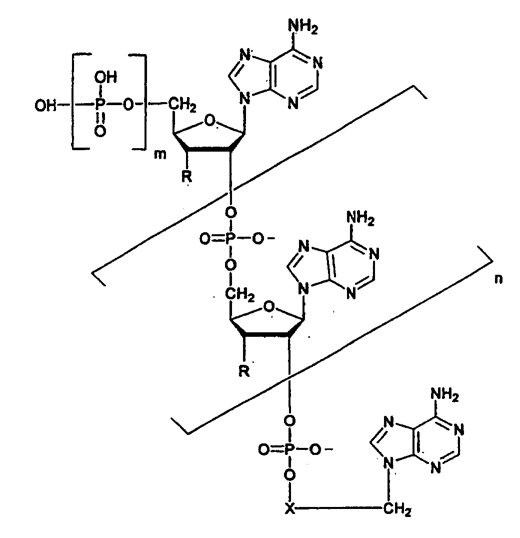 wherein
n is a whole positive integer from 1 to 8,
m is 0, 1, 2 or 3,
R is hydroxyl,
X is selected from C₁ to C ₆ alkylene and C ₁ to C ₆ alkyleneoxy, in the manufacture of a medicament for inhibiting viral infection in a mammal.

33. Use according to claim 32, wherein X is selected from C₁ to C₃ alkylene and C₁ to C₃ alkyleneoxy and n is 1 to 3.

34. Use according to claim 33, wherein X is alkyleneoxy.

35. Use according to any of claims 32 to 34, wherein m is zero.

## Patentansprüche

1. Verbindung der Formel 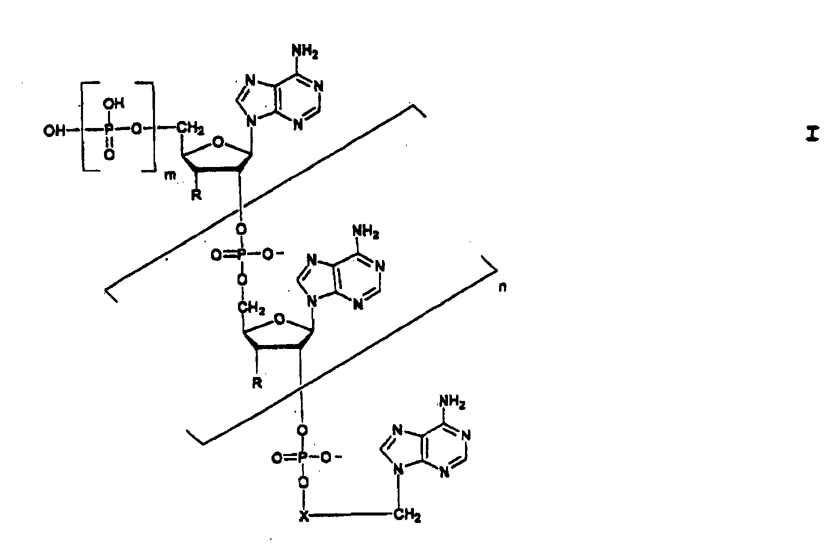 worin
n eine positive ganze Zahl von 1 bis 8 ist,
m 0, 1, 2 oder 3 ist,
R Wasserstoff ist,
X aus C₁- bis C₆-Alkylen und C₁- bis C₆-Alkylenoxy ausgewählt ist, oder ein pharmazeutisch geeignetes Salz davon.

2. Verbindung nach Anspruch 1, worin X aus C₁- bis C₃-Alkylen und C₁- bis C₃-Alkylenoxy ausgewählt ist.

3. Verbindung nach Anspruch 1, die ausgewählt ist aus 3'-Desoxyadenylyl-(2'-5')-3'-desoxyadenylyl-(2'-2')-9-(2'hydroxyethyl)adenin, dessen 5'-Mono-, Di- und Triphosphaten und deren pharmazeutisch geeigneten Salzen.

4. Verbindung nach Anspruch 1, die ausgewählt ist aus 3'-Desoxyadenylyl-(2'-5')-3'-desoxyadenylyl-(2'-3')-9-(3'hydroxypropyl)adenin, dessen 5'-Mono-, Di- und Triphosphaten und deren pharmazeutisch geeigneten Salzen.

5. Verbindung nach Anspruch 1, die ausgewählt ist aus 3'-Desoxyadenylyl-(2'-5')-3'-desoxyadenylyl-(2'-4')-9-(4'hydroxybutyl)adenin, dessen 5'-Mono-, Di- und Triphosphaten und deren pharmazeutisch geeigneten Salzen.

6. Verbindung nach Anspruch 1, die ausgewählt ist aus 3'-Desoxyadenylyl-(2'-5')-3'-desoxyadenylyl-(2'-2')-9-[(2''-hydroxyethoxy)methyl]adenin, dessen 5'-Mono-, Diund Triphosphaten und deren pharmazeutisch geeigneten Salzen.

7. Pharmazeutische Zusammensetzung mit einer Verbindung nach einem der Ansprüche 2 bis 6 und einem pharmazeutischen Träger.

8. Verbindung nach einem der Ansprüche 1 bis 6 zur Verwendung in der Medizin.

9. Verwendung einer Verbindung nach einem der Ansprüche 1 bis 6 bei der Herstellung eines Medikaments zur Verhinderung einer Virusinfektion bei einem Säugetier.

10. Konjugat mit einer Verbindung der Formel 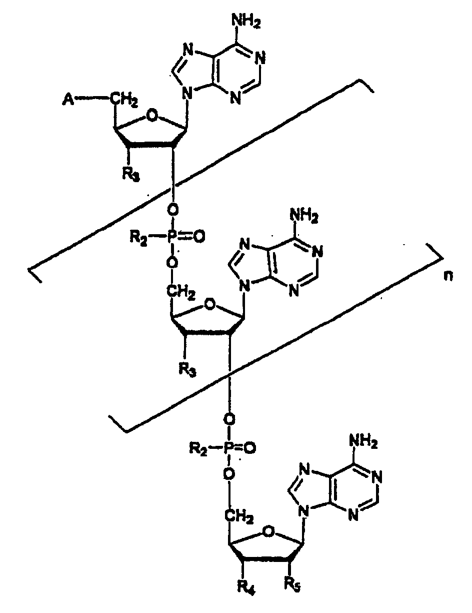 worin
A 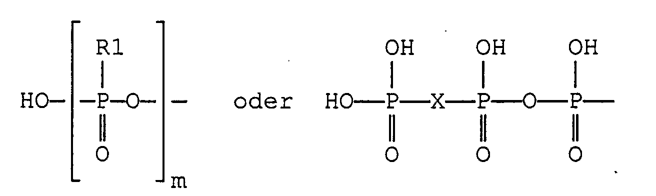 ist,
X NH oder CH₂ ist,
m 0, 1, 2 oder 3 ist,
n eine ganze Zahl von 1 bis 8 ist,
jeder Rest R₁ unabhängig ausgewählt ist aus O⁻, S⁻, Sulfat, Se⁻, C₁- bis C₈-Alkyl und C₁- bis C₈-Alkoxy, jeder Rest R₂ unabhängig ausgewählt ist aus O⁻, S⁻, Sulfat, Se⁻, C₁- bis C₈-Alkyl und C₁- bis C₈-Alkoxy, jeder Rest R₃ unabhängig ausgewählt ist aus Wasserstoff, Hydroxyl, Amino und -OSi(CH₃)₂C(CH₃)₃,
R₄ und R₅ unabhängig ausgewählt sind aus Wasserstoff, Hydroxyl, Amino, C₁- bis C₈-Alkyl, C₁- bis C₈-Alkyloxy, C₁- bis C₈-Alkylamino, Alkylcarbonyl, Alkylcarboxyl und Haloalkyl und C₁- bis C₈-Alkoxyamino, Alkoxycarbonyl, Alkoxycarboxyl und Haloalkoxy oder deren pharmazeutisch geeigneten Salzen,
ausgenommen authentisches 2',5'-Oligoadenylat und dessen Salze,
wobei diese Verbindung über einen Hydroxylsauerstoff in 2'- oder 3'-Stellung ihres 2'-terminalen Nucleotids kovalent an ein Addukt gebunden ist, welches ausgewählt ist aus (i) Vitaminen, die entsprechende Rezeptoren an der Zelloberfläche zur rezeptorvermittelten Endozytose haben, (ii) Addukten mit der Cholesterylgruppe und (iii) Acylgruppen der Formel -CO(CH₂)ₓCH₃, worin
x eine ganze Zahl von 1 bis 20 ist
und wobei das Addukt zu einem verstärkten Eindringen der Verbindung in intakte Zellen führt.

11. Konjugat nach Anspruch 10, worin das Addukt Vitamin B₁₂ ist.

12. Konjugat nach Anspruch 10, worin das Addukt Biotin ist.

13. Konjugat nach Anspruch 10, worin das Addukt Folsäure ist.

14. Konjugat nach Anspruch 10, worin das Addukt lipophil ist.

15. Konjugat nach Anspruch 10, worin
A 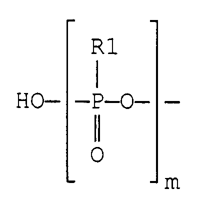 ist,
m 0, 1, 2 oder 3 ist,
n eine ganze Zahl von 1 bis 8 ist,
jeder der Reste R₃ und R₄ unabhängig aus Wasserstoff und
Hydroxyl ausgewählt ist,
jeder der Reste R₂ unabhängig aus S⁻ und O⁻ ausgewählt ist,
R₅ Hydroxyl ist,
oder ein pharmazeutisch geeignetes Salz davon, wobei die Verbindung über die 2'-Stellung ihres 2'-terminalen Nucleotids kovalent an das Addukt gebunden ist.

16. Konjugat nach Anspruch 15, worin das Addukt ein Vitamin ist.

17. Konjugat nach Anspruch 16, worin das Vitamin aus der aus B₁₂, Folsäure und Biotin bestehenden Gruppe ausgewählt ist.

18. Konjugat nach Anspruch 15, worin das Addukt lipophil ist.

19. Konjugat nach Anspruch 15, worin das Addukt eine Cholesterylgruppe enthält.

20. Konjugat nach Anspruch 18, worin das Addukt eine Acylgruppe der Formel -CO(CH₂)ₓCH₃ ist, wobei x eine ganze Zahl von 1 bis 20 ist.

21. Konjugat nach Anspruch 20, worin das Addukt Palmitoyl ist.

22. Pharmazeutische Zusammensetzung, enthaltend eine Verbindung der Formel 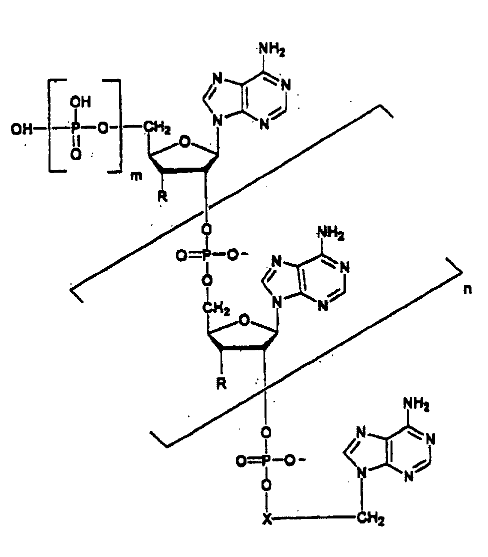 worin
n eine positive ganze Zahl von 1 bis 8 ist,
m 0, 1, 2 oder 3 ist,
R unabhängig Wasserstoff oder Hydroxyl ist,
X ausgewählt ist aus C₁- bis C₆-Alkylen und C₁- bis C₆-Alkylenoxy,
oder ein pharmazeutisch geeignetes Salz davon, und einen pharmazeutischen Träger.

23. Zusammensetzung nach Anspruch 22, worin X ausgewählt ist aus C₁- bis C₃-Alkylen und C₁- bis C₃-Alkylenoxy und n 1 bis 3 ist.

24. Zusammensetzung nach Anspruch 22, worin X Ethylenoxy ist.

25. Zusammensetzung nach einem der Ansprüche 22 bis 24, worin R Wasserstoff ist.

26. Zusammensetzung nach einem der Ansprüche 22 bis 24, worin R Hydroxyl ist.

27. Zusammensetzung nach einem der Ansprüche 22 bis 26, worin m Null ist.

28. Verbindung der Formel 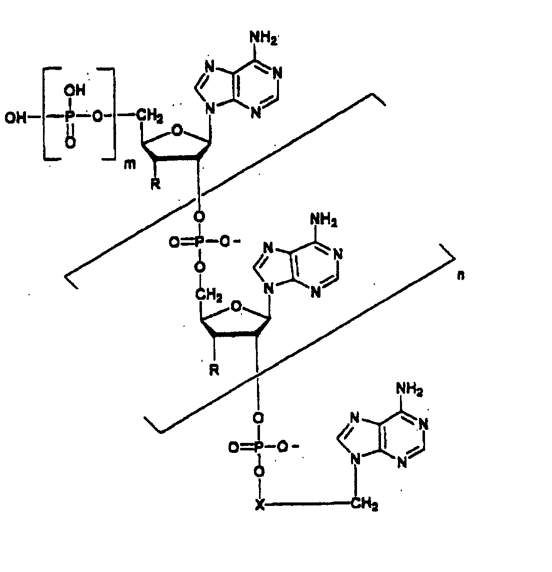 worin
n eine positive ganze Zahl von 1 bis 8 ist,
m 0, 1, 2, oder 3 ist,
R Hydroxyl ist,
X ausgewählt ist aus C₁- bis C₆-Alkylen und C₁- bis C₆-Alkylenoxy zur Verwendung in der Medizin.

29. Verbindung nach Anspruch 28, worin X ausgewählt ist aus C₁- bis C₃-Alkylen und C₁- bis C₃-Alkylenoxy und n 1 bis 3 ist.

30. Verbindung nach Anspruch 29, worin X Ethylenoxy ist.

31. Verbindung nach einem der Ansprüche 28 bis 30, worin m Null ist.

32. Verwendung einer Verbindung der Formel 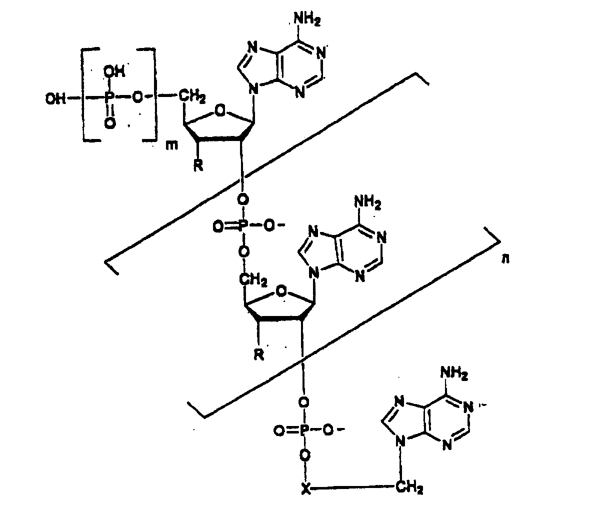 worin
n eine positive ganze Zahl von 1 bis 8 ist,
m 0, 1, 2 oder 3 ist,
R Hydroxyl ist,
X ausgewählt ist aus C₁- bis C₆-Alkylen und C₁- bis C₆-Alkylenoxy,
bei der Herstellung eines Medikaments zur Verhinderung einer Virusinfektion bei einem Säugetier.

33. Verwendung nach Anspruch 32, worin X ausgewählt ist aus C₁- bis C₃-Alkylen und C₁- bis C₃-Alkylenoxy und n 1 bis 3 ist.

34. Verwendung nach Anspruch 33, worin X Alkylenoxy ist.

35. Verwendung nach einem der Ansprüche 32 bis 34, worin m Null ist.

## Revendications

1. Composé selon la formule 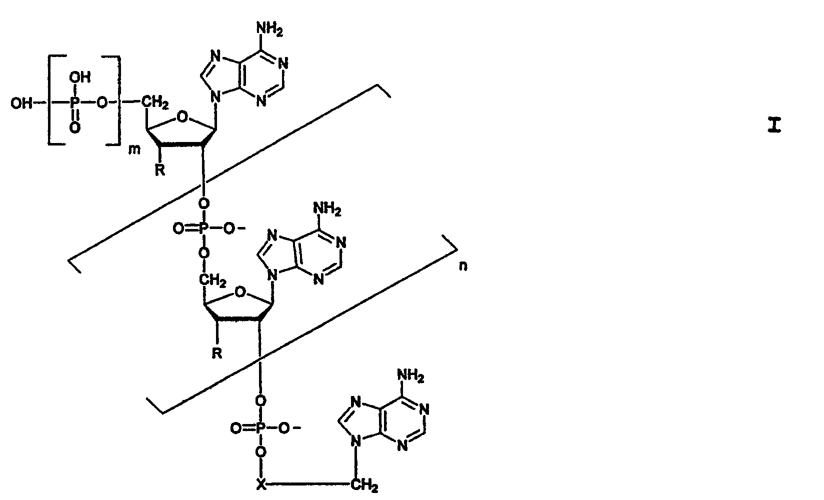 dans laquelle
n est un nombre entier positif de 1 à 8,
m est 0, 1, 2 ou 3,
R est un hydrogène,
X est choisi parmi un alkylène en C₁ à C₆ et un alkylèneoxy en C₁ à C₆, ou un sel pharmaceutiquement acceptable de ceux-ci.

2. Composé selon la revendication 1,
**caractérisé en ce que**
X est choisi parmi un alkylène en C₁ à C₃ et un alkylèneoxy en C₁ à C₃.

3. Composé selon la revendication 1
**caractérisé en ce que**
le composé est choisi parmi la 3'-désoxyadénylyl-(2'-5')-3'-désoxyadénylyl(2'-2')-9-(2'-hydroxyéthyl)adénine, les 5'-mono-, di- et tri-phosphates de celle-ci, et tous sels pharmaceutiquement acceptables de chacun d'entre eux.

4. Composé selon la revendication 1,
**caractérisé en ce que**
le composé est choisi parmi la 3'-désoxyadénylyl-(2'-5')-3'-désoxyadénylyl(2'-3')-9-(3'-hydroxypropyl)adénine, les 5'-mono-, di- et tri-phosphates de celle-ci, et tous sels pharmaceutiquement acceptables de chacun d'entre eux.

5. Composé selon la revendication 1
**caractérisé en ce que**
le composé est choisi parmi la 3'-désoxyadénylyl-(2'-5')-3'-désoxyadénylyl(2'-4')-9-(4'-hydroxybutyl)adénine, les 5'-mono-, di- et tri-phosphates de celle-ci, et tous sels pharmaceutiquement acceptables de chacun d'entre eux.

6. Composé selon la revendication 1
**caractérisé en ce que**
le composé est choisi parmi la 3'-désoxyadénylyl-(2'-5')-3'-désoxyadénylyl(2'-2')-9-[(2"-hydroxyéthoxy)méthyl] adénine, les 5'-mono-, di- et tri-phosphates de celle-ci, et tous sels pharmaceutiquement acceptables de chacun d'entre eux.

7. Composition pharmaceutique comprenant un composé selon l'une quelconque des revendications 2 à 6 et un excipient pharmaceutique.

8. Composé selon l'une quelconque des revendications 1 à 6 pour l'utilisation en médecine.

9. Utilisation d'un composé selon l'une quelconque des revendications 1 à 6 pour la fabrication d'un médicament pour inhiber l'infection virale chez un mammifère.

10. Conjugué comprenant un composé selon la formule 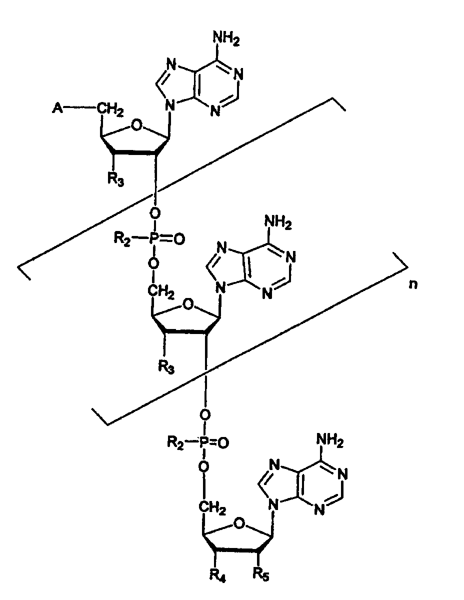 dans laquelle
A est 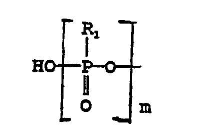 ou 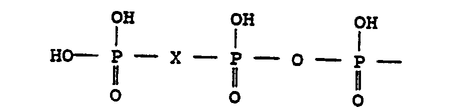
X est NH ou CH₂ ;
m est zéro, 1, 2 ou 3 ;
n est un entier de 1 à 8 ;
chaque R₁ est indépendamment choisi parmi O⁻, S⁻, sulfate, Se⁻, alkyle en C₁ à C₈ et alkoxy en C₁ à C₈ ;
chaque R₂ est indépendamment choisi parmi O⁻, S⁻, un sulfate, Se⁻, un alkyle en C₁ à C₈ et un alkoxy en C₁ à C₈ ;
chaque R3 est indépendamment choisi parmi l'hydrogène, l'hydroxyle, un groupement amino et OSᵢ (CH₃)₂ C(CH₃)₃;
R₄ et R₅ sont indépendamment choisis parmi l'hydrogène, l'hydroxyle, un groupement amino; un alkyle en C₁ à C₈ ; un alkoxy en C₁ à C₈ ;, un alkylamino en en C₁ à C₈ ; un alkoxycarbonyl, un alkoxycarboxyl et halogénoalkoxy ; ou les sels pharmaceutiquement acceptables de ceux-ci, excluant le 2',5'-oligoadénylate authentique et ses sels,
ce composé étant lié de manière covalente à un produit d'addition par un oxygène hydroxyle 2'- ou 3'- du nucléotide 2'-terminal du composé, lequel produit d'addition est choisi parmi (i) des vitamines qui ont des récepteurs correspondant à la surface des cellules pour l'endocytose régulée par des récepteurs, (ii) des produits d'addition contenant le groupe cholestéryle, et (iii) des groupes acyles de formule 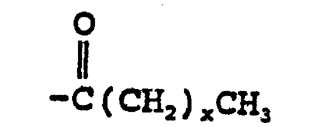 dans laquelle x est un entier de 1 à 20, et lequel produit d'addition engendre une augmentation de la pénétration du composé dans les cellules intactes.

11. Conjugué selon la revendication 10,
**caractérisé en ce que**
le produit d'addition est la vitamine B₁₂.

12. Conjugué selon la revendication 10,
**caractérisé en ce que**
le produit d'addition est la biotine.

13. Conjugué selon la revendication 10,
**caractérisé en ce que**
le produit d'addition est l'acide folique.

14. Conjugué selon la revendication 10,
**caractérisé en ce que**
le produit d'addition est lipophile.

15. Conjugué selon la revendication 10,
**caractérisé en ce que**
le produit d'addition est un groupe acyle de formule 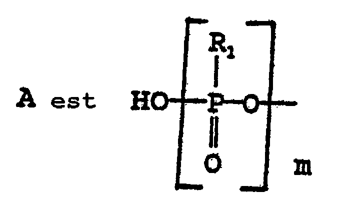 m vaut 0, 1, 2 ou 3
n est un nombre entier de 1 à 8,
chaque R₃ et R₄ est choisi indépendamment entre l'hydrogène et un hydroxyle,
chaque R₂ est choisi indépendamment entre S⁻ et O⁻,
R₅ est un hydroxy ou un sel de celui-ci pharmaceutiquement acceptable,
le composé étant lié en covalence au produit d'addition par la position 2' de son nucléotide 2'-terminal.

16. Conjugué selon la revendication 15,
**caractérisé en ce que**
le produit d'addition est une vitamine.

17. Conjugué selon la revendication 16,
**caractérisé en ce que**
la vitamine est choisie parmi le groupe constitué de la B12, l'acide folique et la biotine.

18. Conjugué selon la revendication 15,
**caractérisé en ce que**
le produit d'addition est lipophile.

19. Conjugué selon la revendication 15,
**caractérisé en ce que**
le produit d'addition contient un groupe cholestéryle.

20. Conjugué selon la revendication 18,
**caractérisé en ce que**
le produit d'addition est un groupe acyle de formule 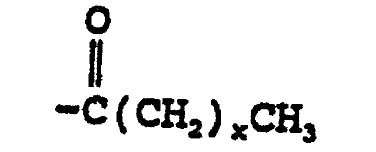 dans laquelle X est un entier de 1 à 20.

21. Conjugué selon la revendication 20,
**caractérisé en ce que**
le produit d'addition est le palmitoyle.

22. Composition pharmaceutique comprenant un composé selon la formule 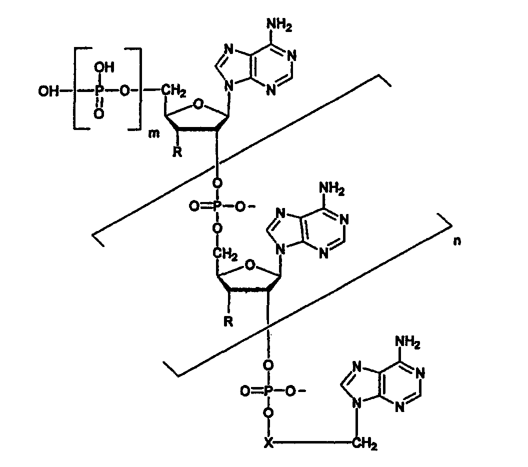 dans laquelle
n est un nombre entier positif de 1 à 8,
m est 0, 1, 2 ou 3,
R est indépendamment un hydrogène ou un hydroxyle,
X est choisi parmi un alkylène en C₁ à C₆ et un alkylèneoxy en C₁ à C₆, ou un sel pharmaceutiquement acceptable de celui-ci, et un excipient pharmaceutique.

23. Composition pharmaceutique selon la revendication 22,
**caractérisée en ce que**
X es choisi parmi un alkylène en C₁ à C₃ et un alkylèneoxy en C₁ à C₃ et n est 1 à 3.

24. Composition selon la revendication 23,
**caractérisée en ce que**
X est un éhtylènoxy

25. Composition selon l'une quelconque des revendications 22 à 24,
**caractérisée en ce que**
R est un hydrogène.

26. Composition selon l'une quelconque des revendications 22 à 24,
**caractérisée en ce que**
R est un hydroxyle.

27. Composition selon l'une quelconque des revendications 22 à 26, dans laquelle m est zéro.

28. Composé selon la formule 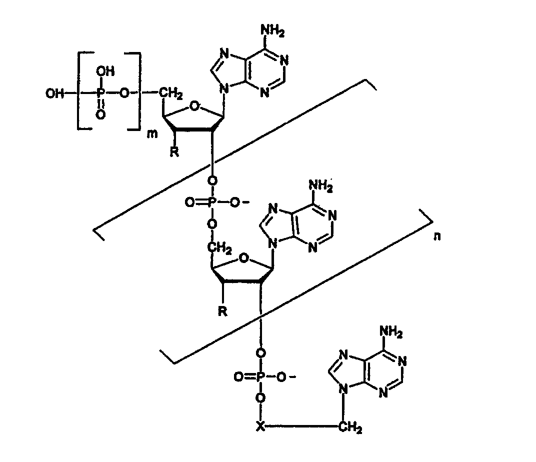 dans la quelle
n est un nombre entier positif de 1 à 8,
m est 0, 1, 2 ou 3,
R est un hydroxyle,
X est choisi parmi un alkylène en C₁ à C₆ et un alkylènoxy en C₁ à C₆,
pour l'utilisation en médecine.

29. Composé selon la revendication 28,
**caractérisé en ce que**
X est choisi parmi un alkylène en C₁ à C₃ et un alkylènoxy en C₁ à C₃ et n est 1 à 3.

30. Composé selon la revendication 29,
**caractérisé en ce que**
X est un éthylènoxy.

31. Composé selon l'une quelconque des revendications 28 à 30, où m est zéro.

32. Utilisation d'un composé selon la formule 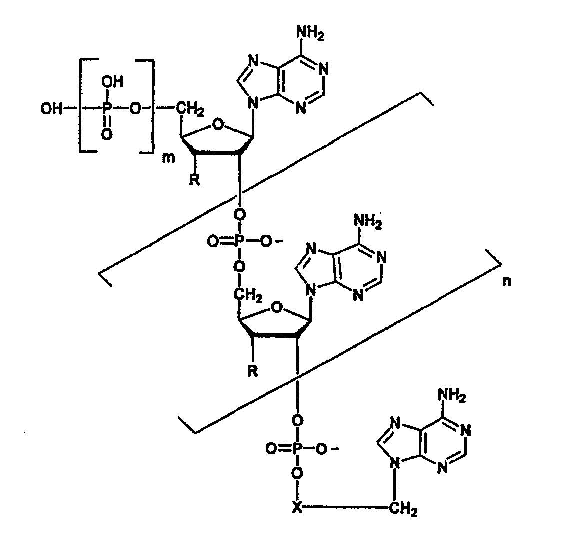 dans la quelle
n est un nombre entier positif de 1 à 8,
m est 0, 1, 2 ou 3,
R est un hydroxyle,
X est choisi parmi un alkylène en C₁ à C₆ et un alkylènoxy en C₁ à C₆,
pour la fabrication d'un médicament pour inhiber l'infection virale chez un mammifère.

33. Utilisation selon la revendication 32,
**caractérisée en ce que**
X est choisi parmi un alkylène en C₁ à C₃ et un alkylènoxy en C₁ à C₃ et n est 1 à 3.

34. Utilisation selon la revendication 33,
**caractérisée en ce que**
X est un alkylenoxy.

35. Utilisation selon l'une quelconque des revendications 32 à 34,
**caractérisée en ce que**
m est zéro.
